# EUROPEAN PATENT SPECIFICATION

(11) **EP 3 134 162 B1**
(45) Date of publication and mention of the grant of the patent: **17.02.2021**
(21) Application number: 15783081.1
(22) Date of filing: 21.04.2015
(51) Int. Cl.: A61M 37/00, A61B 17/00, A61B 17/54, A61B 18/00, A61N 1/04, A61N 1/32

(54) **APPARATUS FOR TRANSDERMAL FLUID DELIVERY**
VORRICHTUNG ZUR TRANSDERMALEN FLÜSSIGKEITSFREISETZUNG
APPAREIL D'ADMINISTRATION TRANSDERMIQUE DE FLUIDE

(30) Priority: 21.04.2014 US 201414257288
(43) Date of publication of application: 01.03.2017
(73) Proprietor: Chang, Franklin, J., San Gabriel, CA 91776 (US)
(72) Inventor: Chang, Franklin, J., San Gabriel, CA 91776 (US)
(74) Representative: Sach, Greg Robert
(86) International application number: PCT/US2015/026834
(87) International publication number: WO 2015/164348

(56) References cited:
- WO-A1-2014/004644
- FR-A1- 2 845 586
- US-A1- 2008 139 995
- US-A1- 2010 049 177
- US-A1- 2010 130 972
- US-A1- 2011 009 882
- US-A1- 2013 137 951
- US-A1- 2013 158 547
- US-A1- 2013 345 661
- US-B1- 6 200 281

## Description

### Background

### 1. Field of the Invention

The present invention relates to a skin treatment tool. More particularly, the present invention relates to an apparatus for transdermal fluid delivery.

### 2. Discussion of the Related Art

Current techniques for superficial skin resurfacing, known as microdermabrasion, treat the outer epidermal layer of the skin by removing the superficial layer to induce the body's own natural wound healing response. It is known in the art to couple microdermabrasion with fluid delivery to enhance therapeutic effects. However, combined microdermabrasion/fluid delivery treatments are hindered by the protective barrier function of the stratum corneum which limits the depth of penetration and absorption to the surface of the skin when drugs and/or fluids are applied to the skin.

Other techniques for skin enhancing include transdermal drug delivery employing an electrical current (e.g., skin electroporation) are known. However, these techniques have limited results based on: 1) the lack of an efficient fluid supply/return system using a vacuum; 2) the impedance of the stratum corneum which limits the efficacy of the current technologies of electrical penetration of drugs and/or fluids; and 3) the optimal permeation structure of the skin occurs during application of an electrical current and only lasts a few seconds after application of the electrical pulse.

Known technologies for delivery of an electro-current to the skin suffer from one or more of the following deficiencies which lead to limited results, including, a lack of an efficient fluid supply/return system using a vacuum source; an inability to simultaneously apply fluid and electro-current to the skin; as a means to lower the impedance of the stratum corneum.

The major disadvantage of the conventional art is that the fluid cannot be directly applied from on the abrading surface to the skin. An injection end of a tube is extended close but separate to the abrading surface so that the fluid is injected to the abrading surface through the injection end. The fluid flowing through injection end will not be evenly distributed the fluid on the abrading surface when applying on the skin. Most of the fluid in fact will never be in contact with the skin and be wasted because the fluid cannot fully penetrate between the skin and the abrading surface of the skin. More importantly, the individual injection tube structure is used when there is a motor utilized in the microdermabrasion device.

US. Pub. No. 2010/0049177 A1, Boone, discloses a microdermabrasion system which comprises a tip having an abrading surface and a side surface, wherein a plurality of fluid channels terminate on the side surface of the tip. That is to say, the fluid cannot be directly delivered through to the abrading surface of the tip. Boone further discloses a plurality of radiation sources evenly distributed around a perimeter of the tip and between the tip and the vacuum opening. This structure has a major disadvantage that the fluid will only deliver to the radiation sources but not the abrading surface because of the vacuum effect at the vacuum opening. Therefore, the user must hold the hand piece of Bonne to manually move to the tip on the skin. Also, Boone describes using radio frequency to heat below the skin but does not describe any relationship to the fluid delivery or abrasive. It is a means to penetrate heat into deeper layers to cause tightening of the skin but do not create a transdermal pathway for fluid. This type of frequency also has no relationship with abrasion and liquid.

US. Pub. No. 2004/0138680 A1, Twitchell et al., disclosed a microdermabrasion apparatus comprising an exfoliation tip mechanically coupled to a motor via a shaft and a tube extended to a vacuum pace in the suction cup. The suction cup as taught by Twitchell et al. is arranged in such a way that the user's skin is pulled partially into the suction cup where a vacuum is formed within the space in the suction cup. That is to say, no fluid is applied onto the exfoliation tip and no fluid is sucked back via the tube.

US. Pat. No. 8,343,116, Ignon et al., disclosed a skin treatment system comprising a tip having at least one abrasive element configured to abrade skin, a delivery port and a suction port extended to a working surface of the tip, wherein the delivery port delivers fluid from a first canister to the working surface of the tip and the suction port sucks the fluid back to a second canister from the working surface of the tip. The disadvantage of the system as taught by Ignon et al. is that the fluid will be sucked back by the suction port right after the fluid is delivered to the working surface of the tip. That is to say, the fluid will not be applied long enough on the working surface of the tip. Also, without any motor incorporated within the system as taught by Ignon et al., the user must hold the hand piece of Ignon et al. to manually move the tip over the skin in a scratching motion. Thus, the system as taught by Ignon et al. makes it impossible to incorporate with any electrodes because both the delivery port and suction port are located right at the working surface of the tip. So the fluid cannot be delivered to be in contact with any electrode after it is vacuumed back by the suction port.

FR284556 (FR56) discloses a device for treating skin cosmetically by abrasion and spraying a liquid under pressure, and WO 2014/004644 discloses a skin treatment device. As described in FR56, which may be regarded as a closest prior art, the interior components of the device of FR56 are held within the walls of the handle (1) of that device with no other supporting structure such as a casing between those interior components and the handle (1). In FR56 device both the fluid inlet pipe (3) and the fluid outlet conduit (6) have their entry and exit ports respectfully on one side of the handle (1). The FR56 device has a multi-functional tip having a skin-applying surface, a support member within a handle structure, comprising a fluid delivery structure, a fluid returning structure and a tip driver. The skin applying surface of the multifunctional tip comprises a plurality of abrading elements. The support member comprising the fluid delivery structure, the fluid returning structure and the tip driver is secured inside a casing within handle structure. The tip driver comprises, a driving unit and a driving shaft, wherein the driving shaft is operatively extended from the driving unit to the multifunctional tip through a center slot of the support member, wherein the driving shaft is movable and the support member is stationary. The multifunctional tip is detachably coupled at a free end of the driving shaft, wherein the shaft has a hollow portion extended to the multi-functional tip, wherein a fluid delivery channel of the fluid delivery structure is defined at the hollow portion of the driving shaft, wherein the fluid delivery structure further has a fluid inlet transversely formed at the driving shaft to guide a fluid from an interior fluid cavity of the support member into the fluid delivery channel defined at the hollow portion of the driving shaft; wherein the fluid delivery structure has an aperture located at an axial center of said the applying surface of the multi-functional tip to communicate with the fluid delivery channel defined at the hollow portion of the driving shaft and deliver fluid to the skin surface; wherein the driving shaft has a dual function of the skin applying surface of the multi-functional tip to rotate while concomitantly guiding a flow of a fluid through the fluid delivery channel to the skin applying surface of the multi-functional tip at the aperture; wherein the driving unit is operated to generate a movement by a motor at the skin applying surface of the multi-functional tip through the driving shaft and wherein the fluid returning structurecomprises a vacuum inlet, a vacuum port and a vacuum passage, wherein the vacuum passage is extended through the support member to transport a used fluid collected by the vacuum inlet from the multifunctional tip by the vacuum port to a reservoir at a rear end of the support member.

Accordingly, there is a need for a skin resurfacing and enhancement system with an enhanced fluid delivery/fluid return capacity which also improves the permeation structure of the skin. The present invention discloses an apparatus for transdermal fluid delivery which provides three different skin treating functions for skin treatment to transdermally penetrate fluid deeper into the skin by means of simultaneous 1) abrasive peeling 2) electrical stimulation 3) liquid infusion in order to improve the skin structure affecting multiple layers of the skin, such as the epidermis, dermis, and hypodermis. The present invention also provides an innovative structure to simultaneously guide the fluid to the tip surface and to prolong the traveling path of the fluid.

### Brief Summary of the Invention

According to the present invention, a device for a combination treatment of the top and bottom layer of a skin surface are described. The device comprises features according to claim 1.

A method of treating the skin to transdermally penetrate fluid deeper into the skin by means of simultaneous 1) abrasive peeling 2) electrical stimulation 3) liquid infusion to improve the skin structure affecting multiple layers of the skin, such as, epidermis, dermis, and hypodermis is also disclosed, however does not form part of the present invention. The apparatus of the present invention serves as an all-in-one handheld skin treatment device.

The device and methods described herein allow for the simultaneous deep penetration of fluid through the skin by applying an electric current and an abrasive media in the working end of the device to increase skin's permeability. Techniques known as electroporation, ultrasound, and other electrical induced therapies, etc., which use electric currents to go deeper past the stratum corneum to stimulate cells underneath the skin may be employed in the device. The combination of the electrical induced therapies and microdermabrasion create aqueous pathways to increase the permeability of the drugs and/or fluids which are delivered from a supply and return reservoir by a vacuum system within the device. A pressure mechanism may also be employed as part of the device.

In a preferred embodiment, the tip of the device has an outer structure having one or more electrodes and an intermediate structure having an abrading end portion, where the abrading end portion has an abrasive media. The tip of the device also has an inner structure which has one or more apertures for fluid delivery. That is to say, the inner structure is located at the center of the tip. The outer structure is located at the periphery of the tip. The intermediate structure is located between the inner structure and the outer structure. The outer structure, intermediate structure, and the inner structure are coaxial with each other and are in a ring shape. Preferably, the outer structure and intermediate structure form an outer ring and intermediate ring respectively at the tip. The outer ring and intermediate ring can be formed in a circular shape or a non-circular shape. Therefore, the abrading end portion forms at the intermediate ring and encircles the fluid delivery. The electrodes are aligned at the outer ring to encircle the abrading end portion at the intermediate ring. Preferably, at least one of the structures is removable, and more preferably, each of the outer structure, intermediate structure, and inner structure are removable, and most preferably, at least one of the structures is disposable.

A method for treating a skin surface of a patient is disclosed, but does not form part of the invention. According to the method, first an abrasion device for treating a skin surface of a patient is selected, wherein the abrasion device comprises one or more electrodes, an abrading end portion having an abrasive media, and one or more apertures for fluid delivery. Next, the abrading end portion of the device is placed on the skin surface of the patient. The patient's skin is then treated by applying the abrasive media to the skin surface of the patient, delivering fluid to the skin surface of the patient, and applying an electrical current to the skin surface of the patient. The patient skin is treated with abrasive media, fluid delivery, and current delivery in the order stated above, simultaneously, or another order. Vacuum may then be applied to the skin surface of the patient.

A kit for treating a skin surface of a patient is also described. The kit comprises a skin abrading device comprising a tip, wherein the tip has at least one current delivery tip having one or more electrodes, a plurality of abrading tips, wherein each abrading tip has an end portion with an abrasive media, and wherein the plurality of abrading tips are removable from the device and interchangeable, and a fluid delivery tip having one or more apertures for fluid delivery. Preferably, the tip further comprises a vacuum entry port and also preferably, each of the plurality of abrading tips has a grit size, and the grit size varies for each abrading tip.

According to the invention a device for treating a skin surface of a patient, comprises a multi-functional tip having a skin applying surface, a fluid delivery structure, and a tip driver.

The tip driver comprises a driving unit and a driving shaft operatively extended from the driving unit to the multi-functional tip, so that the driving unit is operated to generate a movement at the skin applying surface of the multi-functional tip. The driving shaft has at least a hollow portion extended to the multi-functional tip.

The fluid delivery structure is arranged to directly guide a flow of fluid on the skin applying surface of the multi-functional tip. The fluid delivery structure has a fluid channel defined at the hollow portion of the driving shaft and at least an aperture formed at the skin applying surface of said multi-functional tip to communicate with the fluid channel. Therefore, the driving shaft provides multifunction of driving the skin applying surface of the multi-functional tip to rotate and guides the fluid through the fluid channel to the skin applying surface of the multi-functional tip at the aperture at the same time.

For a more complete understanding of the present invention with its objectives and distinctive features and advantages will be described herein below.

### BRIEF DESCRIPTION OF THE DRAWINGS

These and other features, aspects and advantages of the present invention will become better understood from the following description, appended claims, and accompanying figures where:
**Figure 1A** shows an exemplary skin abrading device 100 which does not form an embodiment of the present invention.
**Figure 1B** is partial side cut-away view of the device 100, shown in Figure 1A.
**Figure 2A** is a top perspective view of the device 100, shown in Figure 1A and Figure 1B, showing the tip 104 of the device 100.
**Figure 2B** and **Figure 2C** are alternate examples for the tip 104 of the device 100, which do not form an embodiment of the present invention.
**Figure 3A** is a side view of one embodiment of the device 100, having a plurality of removable, exchangeable, and attachable tips.
**Figure 3B** is a side view of another example of one of the tips shown in Figure 3A.
**Figure 4** is a partial side cut-away view of the device 100 having a wide-angle tip 104 which does not form an embodiment of the present invention.
**Figure 5A** is a side view of another example of the device 100, having a plurality of removable, exchangeable, and attachable tips, where the electrodes 108a, 108b, are concentric circles,which does not form an embodiment of the present invention.
**Figure 5B** is a partial side cut-away view of the device 100 shown in Figure 5A, having electrodes 108a, 108b, which are concentric circles.
**Figure 6A** shows an alternate example of the skin abrading device 100 which does not form an embodiment of the present invention, having a divided handle 102a and 102b.
**Figure 6B** is a cut-away view showing the divided handle 102a and 102b of Figure 6A.
**Figure 7** is a perspective view of the tip detachably coupling at the handle which does not form an embodiment of the present invention.
**Figure 8** is an exploded view of the tip according to the above example, showing the replacement of the electrode rings.
**Figure 9** is a top view of the tip according to the above example.
**Figure 10** is a top view of the tip according to the above example, showing one electrode ring at the intermediate structure.
**Figure 11** is a top view of the tip according to the above example, showing the alternative of the outer and intermediate structures.
**Figure 12** is a top view of the tip according to the above example, showing how to increase the abrading surface of the tip.
**Figure 13** is a perspective view of the tip detachably coupling at the handle according to another example, which does not form an embodiment of the present invention, showing the electrode skin treating tip.
**Figure 14** is a perspective view of the tip detachably coupling at the handle according to another example, which does not form an embodiment of the present invention, showing the micro-needle skin treating tip.
**Figure 15** is a modification of the micro-needle skin treating tip according to the above example.
**Figure 16** shows an apparatus for transdermal fluid delivery according to the embodiment of the present invention.
**Figure 17** is a sectional view of the apparatus in Figure 16.
**Figure 18** is an exploded view of the driving shaft and the support member of the apparatus according to the present invention.
**Figure 19** shows a modification of the electrode module of the apparatus according to the present invention.

### DETAILED DESCRIPTION OF THE INVENTION

According to the present invention, a device, i.e. a microdermabrasion device, for increasing the permeability of the skins surface to fluid and/or drug delivery is described. In general, permeation of drugs and/or fluids through the skin occurs at a slow rate, if at all. The stratum corneum acts as a barrier that limits the penetration of substances through the skin. Application of high-voltage pulses to the skin increases its permeability (electroporation) and enables the delivery of various substances into and through the skin. The application of electroporation to the skin has been shown to increase transdermal drug delivery. Moreover, electroporation, used alone or in combination with other enhancement methods, expands the range of drugs (small to macromolecules, lipophilic or hydrophilic, charged or neutral molecules) that can be delivered transdermally. The efficacy of transport depends on the electrical parameters and the physicochemical properties of drugs. The in vivo application of high-voltage pulses is well tolerated.

According to the embodiment of the invention, a device according to claim 1 and comprising an abrading surface, fluid delivery, current delivery; and fluid vaccuation is described. The device enhances fluid delivery through the stratum corneum by first delivering an abrasive media to the surface of the skin to prepare the skin for fluid delivery. Next, the device delivers fluid to the surface of the skin, with simultaneous current delivery (electroporation). The combination of skin abrasion, followed by simultaneous fluid delivery with electroporation allows for deep penetration of fluid through the skin by increasing the skin's permeability. In addition to enhancing fluid delivery through the stratum corneum, the device resurfaces the outer surface of the skin, removing dead skin cells and the outer layer of dermis, along with other superficial imperfections. Unlike known microdermabrasion devices, the results achieved with the device of the present invention will have enhanced and longer lasting results, namely, because skin enhancing fluids and drugs are delivered more deeply into the skin with the simultaneous electrooporation, and the electrical induced therapy itself has skin enhancing properties, such as increased collagen production, muscle tone, and overall skin elasticity and firmness.

The device and methods, which do not form part of the present invention, being described herein have an efficient fluid supply/return for transdermal/topical delivery of skin enhancing drugs and medicaments. This feature of the invention has been found to be particularly important since presently known technologies use a gel which is applied to the skin which limits the penetration of effective ingredients because of the greater molecular weight of the gel. Macromolecule delivery through a liquid, which can be accomplished with the present invention, is accordingly more effective than prior art technologies which use a gel. The application of an abrasive as described herein solves this issue of lowering the impedence of the stratum corneum thus further improving drug delivery to the skin. Accordingly, the device of the present invention, which include fluid delivery with electro-current and a vacuum source, enable simultaneous application of fluids containing skin enhancing drugs, with increased topical delivery through an abrading surface, to achieve the maximum effect. The abrading surface, which is applied to the skin preferably prior to fluid/drug delivery, increases topical drug delivery and penetration of the drug to the lower layers of the skin. These features of the invention are an improvement over prior art technologies which lack a fluid delivery and a vacuum source and more particularly in combination with an abrading surface and electro-current application to accomplish skin resurfacing and enhancement.

As used in this disclosure, the term "comprise" and variations of the term, such as "comprising" and "comprises," are not intended to exclude other additives, components, integers or steps.

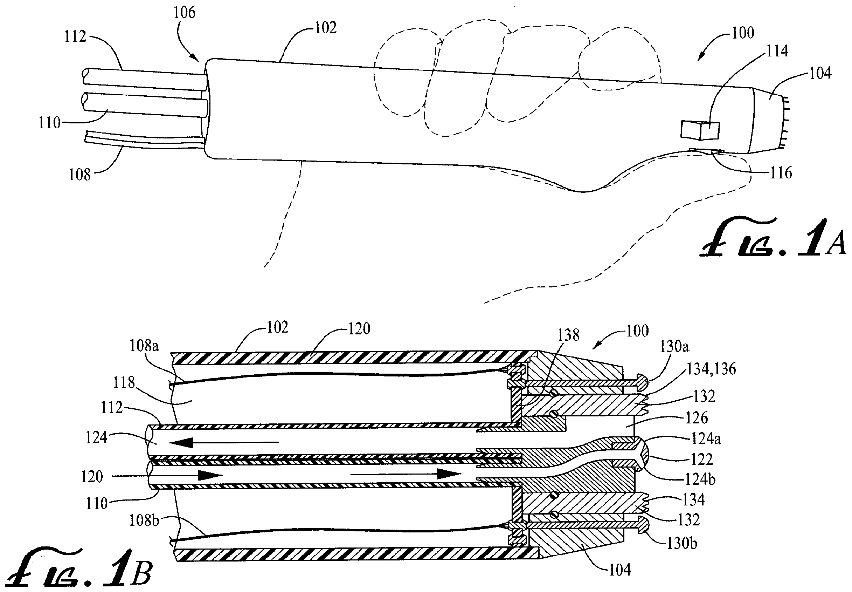

An exemplary device for enhancing fluid delivery to the skin is described. Referring now to **Figure 1A**, a skin abrading device **100** having fluid and current delivery is shown. The device **100** comprises a handle **102**, a tip **104**, and a distal end **106**. Positioned at the distal end are one or more conduits such as an electrical conduit **108**, a fluid delivery conduit **110**, and a vacuum conduit **112**. The skin abrading device **100** may further include one or more switches for controlling the device **100** such as a switch **114** and/or **116** for controlling electrical current delivered via the electrical conduit **108**, and/or control vacuum and/or fluid delivery from the fluid delivery and vacuum conduits **110** and **112**. However, in other examples, these switches are positioned remotely on an adjunct device. The optional vacuum function of the evacuates fluid and skin debris from the surface of the skin and delivers the evacuated fluid and skin debris to an optional waste container (not shown) which may be positioned on the handle or in an adjunct device.

As shown in **Figure 1A****,** the handle **102** may be cylindrical with molded hand grip, or it may have other configurations such as cylindrical (without a molded hand grip), or other variations, including elliptical, square, rectangular, and variations thereof. The handle **102** may be formed of various materials as known to those in the art including any suitable plastic, metals, such as aluminum, stainless steel, and other alloys, and combinations of metal and plastic. Preferably, the handle **102 i**s made from a high density plastic material.

Referring now to **Figure 1B****,** a partial side cut-away view of the device **100** shown in **Figure 1** is shown. As shown in **Figure 1B****,** the handle **102** of the device **100** comprises an interior **118** and an outer casing **120.** The fluid delivery conduit **110** is positioned in the interior **118** of the handle **102** and delivers fluid **120** from a reservoir (not shown) in an adjunct device through the fluid delivery conduit **110** and out the tip **104** of the device **100**. The fluid **120** exits the tip **104** through a fluid delivery tip **122** having one or more apertures **124.** Also positioned within the interior **118** of the handle **102** is the vacuum conduit **112** which pulls a vacuum from a vacuum pump (not shown) stationed in an adjunct device through the vacuum conduit **112.** The vacuum conduit **112** has a vacuum entry port **126** positioned within the tip **104** for evacuating fluids and other debris from the surface of the skin. The interior **118** of the device **100** has one or more electrical conduits **108a, 108b,** which deliver current either to an electronics board **128,** which then delivers current to one or more electrodes **130,** shown as **130a** and **130b.** Positioned within the tip **104** is an abrading structure **132** having an abrading end portion **134,** which comprises an abrasive media **136**. Within either the interior **118** of the device, electronic control circuitry **138** may be positioned for controlling current to the electrodes **130**.

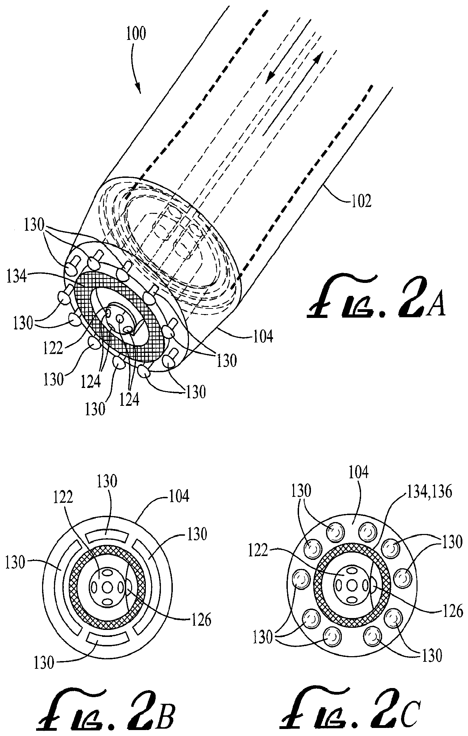

Referring now to **Figures 2A, 2B**, and **2C**, examples of the tip **104** of the device **100** are shown. As shown in Figure **2A****,** the tip **104** may be somewhat tapered at the end, or in other examples, the tip **104** may be substantially cylindrically shaped or other, such as oval shaped, squared, or rectangularly shaped. As also shown in **Figure 2A**, preferably, the fluid delivery tip **122** is domed shaped, having a plurality of apertures 124, such that a spray effect is achieved with the fluid delivery tip **122.** (However, in other examples, the fluid delivery tip **122** may be flat, and/or have a single aperture **124**. Multiple apertures **124** spread the liquid evenly along the area of the skin. Preferably, the fluid delivery tip **122** is positioned with respect to the tip **104,** electrodes **130,** and abrading structure **132** such that the fluid delivery tip extends slightly beyond or substantially flush with the abrading structure **132.**

The tip **122** of the dome creates a planar surface of the skin preventing the vacuum suction from causing a subcutaneous hemotoma which is caused when the lining of blood vessels are damaged and blood escapes through the skin.

The vacuum entry port is positioned with respect to the tip **104,** such that the vacuum entry port **126** minimizes skin trauma and ruptured capillaries, veins and arteries from the vacuum **124,** yet creates a suitable vacuum to evacuate fluid and debris from the skin's surface. According to an example, the vacuum entry port **126** is positioned on the tip **104** such that when the tip **104** of the device **100** is applied to the surface of the skin, a space is created between the tip **104** and the vacuum entry port **126** to create a vacuum, known in the art as a closed loop system.

In another example, the fluid delivery tip **122** is substantially flush to the skin with respect to the abrading end portion **134** of the abrading structure **132** and the electrodes **130** such that when the device **100** is applied to the skin, the skin stays relatively flat during treatment. According to this example, when the abrasive media **136**, vacuum **124**, fluid **120**, and electric current **140** are applied to the skin with the configuration described with respect to this example, having the various structures of the tip **104** substantially flush to the skin minimizes the possibility of skin trauma associated with the pulling up of skin in a space of vacuum **124.**

In another example, the vacuum entry port **126** can be positioned in other portions of the tip **104** to provide an optimal vacuum of concurrent liquid delivery and/or removal of skin debris. However, the vacuum entry port **126** is preferably positioned to keep a higher level of fluid within the tip of the handle during treatment so as to have a higher absorption and penetration rate of ingredients contained in the fluid, into the skin, while still evacuating skin debris and preventing the fluid **120** from flowing away from the desired treatment area and/or falling off the skin.

The abrading structure **132** is positioned with respect to the tip **104,** such that the abrading end portion **134** of the abrading structure **132** is substantially flush to the surface of the skin. In other examples, the abrading structure **132** may be lowered or raised with respect to the end of the tip **104** to provide skin contact, as desired by the user.

In this example, the abrading structure **132** has a range of abrasiveness on the abrasive media **136** from a substantially smooth surface (no abrasion) to very abrasive depending on the treatment type. As shown in **Figures 1B****,** and **2A****-2B,** the abrading structure **132** is positioned on the outer edge of both a fluid supply, i.e., the fluid delivery tip **122** and vacuum port **126** and on the inside of the electrodes **130.** However, other arrangements of the abrading structure **132**, electrodes **130,** and fluid delivery tip 122 and vacuum port **126** are possible, as will be understood by those of skill in the art.

The abrading structure **132** may be reusable or disposable, in part or entirely. For example, the abrading end portion 134 and the abrasive media **136** are integral to the abrading structure **132.** According to this example, the abrading structure may be reusable or disposable in part or entirely. When the abrading structure **132** is reusable, it is preferably designed to be sanitized and cleaned between uses and reused. In an alternate example, the abrasive media **136** is positioned on the abrading end portion 134 in a removable fashion, such as a removable strip. According to this example, the abrading structure **132** is generally reusable and the abrasive media **136** on the abrading end portion **134** is preferably disposable.

The abrasive media **136** comprises a material suitable to abrade the surface of the skin such as sand paper, rough textiles (such as dermal grade fabrics that are used in cosmetic microdermabrasion, typically made from 100% medical grade nylon and have a plurality of coatings and finishes), wire brushes, carbon fibers, and micro-needles. The material can be conductive or non-conductive. According to one example, the abrasive media **136** comprises a non-conductive sand paper. In one embodiment, the sand paper is white aluminum oxide, a non-conductive material, readily available at low cost in medical grade. This material is able to withstand elevated temperatures, such as those typically present in any vitrification process that may be necessary for high volume binding/fabrication to produce the abrasive tip. According to other examples, a material softer than aluminum oxide is preferred so that the material is less irritating to the skin than aluminum oxide. According to this example, the abrading media **136** comprises polymeric beads. Generally, polymeric beads provide a softer, less irritating material than aluminum oxide. However, other materials may be used as the abrading media **136**, where the material is selected based on the particular individual to be treated and the purpose of the treatment. Accordingly, for different individuals, different materials may be substituted for the above-listed materials. In other examples, the abrasive media **136** comprises a conductive material. Suitable conductive materials include, but are not limited to, metals, carbon, conductive polymers and conductive elastomers.

The abrading end portion **134** may have a variety of suitable thicknesses and diameters. According to one example, abrasive particles are coated onto the abrading end portion of the abrading structure **132.** In some examples, the abrading structure **132** and abrading end portion **134** comprise a unitary plastic structure, such as acrylonitrile butadiene styrene (ABS). According to this example, the abrasive media is an abrasive coating adhered to the abrading end portion **132,** or the abrasive media **136** is of a unitary construction with the abrading structure **132** and abrading end portion **134.** According to one example, the abrasive media comprises abrasive particles which are adhered to the abrading end portion **134,** where the thickness of the abrasive media **136** is defined by the grit size of the abrasive particles. According to this example, the abrasive particles are generally of a size ranging from about about 50 to 300 microns (50 to 300 grit), and typically about 163 to 173 microns (100 to 120 grit) and may comprise carborundum (aluminum oxide), sodium bicarbonate, polymeric particles, and the like. Coarser particles (at the lower ends of the grit ranges (about 35 to 50, and typically less than 100), micron ranges (about 439 to 313, an typically less than 163)) may also be provided for use in initial treatments, or treatments on coarser areas of the skin (such as arms), while finer particles (at the higher ends of the grit ranges about 300 (57 microns) and above) may be employed for subsequent treatments. Alternately, the abrading end portion **134** may be formed by knurling, machining, laser treatment or otherwise mechanically or chemically treating the end of the abrading end portion **134** to provide an integral abrasive media **136** which has a unitary construction with the abrading end portion and abrading end structure **132.** In an example, the abrasive media 136 is abrasive particles having a grit size of about 120 or lower (approximately 137 microns in diameter).

Typically the abrading end portion **134** will have a thickness ranging from 0.5 microns to 150 microns, preferably ranging from 15 microns to 120 microns. The diameter of the abrading end portion **134** is variable depending on the type of application. For example, in applications having a small area to be permeabilized, the abrading end portion **134** can have a diameter of up to several micrometers, such as from 1 to 25 microns. For applications having a larger area to be permeabilized, the abrading end portion **134** can have a diameter of up to several inches, such as from 0.1 to 5 inches (2.5 mm to 127 mm).

According to the present example, a current 140 (not shown) is delivered from the device **100** to the surface of the skin through one or more electrodes **130.** The electrodes **130** can be a single electrode, or a plurality of nodes or combination thereof, and may further have a variety of configurations and dimensions, such as nodes, bars, etc., as will be understood by those of skill in the art.

Electrical currents, known for application to the skin, which may be used include:
a. Electroporation. Electroporation refers to the application of electric pulses to increase the permeability of cell membranes. Electric pulses are applied to skin cells to increase membrane permeability.
b. Microcurrent. Microcurrent refers to the application of a small current used in a noninvasive electrotherapy technique where electrodes are applied at acupuncture points. In general, 10-500 microamps (Ua) are applied to the surface of the skin and for optimal effectiveness, the current applied to the skin should not cause an actual "visual" contraction of the facial muscles. In some applications, electroporation refers to the process of applying a microcurrent to the surface of the skin.
c. Iontophoresis. Iontophoresis refers to a therapeutic type of transcutaneous drug delivery in which electric current is applied to the skin to enhance absorption of large polar or hydrophilic molecules and peptides-e.g., insulin, and control therapeutic delivery. A galvanic current is applied an ionizable agent in contact with a surface of the skin, by means of an appropriate electrode, to hasten the movement into the tissue of the ion of opposite charge to that of the electrode. Accordingly, skin enhancing agents which are polar or hydrophilic may be delivered into the skin.
d. Sonophoresis. Sonophoresis refers to a process that exponentially increases the absorption of semisolid topical compounds (transdermal delivery) into the epidermis, dermis and skin appendages. Sonophoresis occurs where ultrasound waves stimulate micro-vibrations within the skin epidermis and increase the overall kinetic energy of molecules making up topical agents. Skin enhancing agents may be mixed with a coupling agent (gel, cream, ointment) to transfer ultrasonic energy from the ultrasound transducer (i.e., electrode) to the skin and enhancing drug transport through the skin.
e. Galvanic. Galvanic or Galvanic current refers to the current which is the electrical current used in the process of Iontophoresis.
f. Ultrasound. Ultrasound or ultrasonic current refers to the current used in Sonophoresis. Ultrasound is cyclic sound pressure with a frequency greater than the upper limit of human hearing. Although this limit varies from person to person, it is approximately 20 kilohertz (20,000 hertz) in healthy, young adults and thus, 20 kHz serves as a useful lower limit in describing the ultrasonic current applied via the electrodes.
g. Ultrasonic Cavitation. Ultrasonic Cavitation refers to an advanced ultrasonic machine having 3 MHz and 1MHz ultrasound frequencies for the body and a 1.4MHz ultrasonic frequency for the face, and an ultrasonic cavitation wavelength at 47 KHz. In Ultrasonic Cavitation, the ultrasonic waves are able to act on the skin surface (3MHZ ultrasound), providing skin tightening as well in the deep layers, (cavitation) providing real results, after the treatment, in terms of cellulite and localized adiposity. It has been shown to be able to eliminate centimetres of belly, buttocks, hips and thighs without any side effects. Ultrasonic waves in a specific range from 20 to 70 KHz are able to cause the "cavitation" effect: focused high energy waves which creates micro bubbles of vapor inside the adiposities and in the interstitial liquids of cellulite.
h. Acoustic Cavitation. Acoustic Cavitation refers to a non-flowing system where the ambient pressure can be varied by sending sound waves through a liquid. The ultrasonic sound waves are made up of alternate compressions and rarefactions. During the rarefaction cycle (low pressure) a lot of microscopic bubbles will grow and during the compression cycle (high pressure) each bubbles undergoes a collapse or implosion.
i. Mesotherapy. Mesotherapy refers to a procedure in which multiple tiny injections of pharmaceuticals, vitamins, etc., are delivered into the mesodermal layer of tissue under the skin, to promote the loss of fat or cellulite.
j. Radio Frequency. Refers to a procedure using a beam of radio frequency energy to target deeper layers of the skin by heating them up. This creates stimulation of the skin and in particular, the collagen, a substance which gives elasticity to the skin. The radio frequencies cause water molecules in the deeper layers of skin to vibrate. This in turn creates friction which causes the heating effect. When heat is applied to collagen fibres, they shrink and tighten up, and over time following the treatment, new collagen also forms.
k. Hot and cold therapies. Refers to using an electrical current and other modalities to create different adjustable temperatures ranging from hot (up to 140 degrees Fahrenheit) to cold (down to 5 degrees Fahrenheit) to treat the surface layer skin by softening and/or tightening collagen fibers.

In an example, a microcurrent is applied to the skin, i.e., electroporation. According to this example, the current of the device 100 is set for a wave form with power between 10-500 microamps (Ua). The current 140 (not shown) is delivered through the device 100 and through one or more electrodes 130 to the surface of the skin. Treatment can be substantially stationary in certain areas, or vary in the degree of motion, up to sweeping lines.

According to another example, a combination of two or more frequencies of current are applied from the device 100 to a patient. Accordingly, in some examples the device is capable of delivering a plurality of different frequencies (i.e., types) of current, either individual applied or concurrent. For example, an ultrasonic current may be applied from the device 100 to a patient, followed by delivery of a microcurrent from the device 100 to the same patient. The treatment may be in one treatment area, or over a plurality of treatment areas, such the delivery of microcurrent to the face, followed by delivery of ultrasonic current to the arms. The plurality of frequencies may be used on one patient for application of different electric currents. For example, ultrasound and microcurrent have different ways of penetrating fluids and treating the skin. The concurrent combination of these and other electric modalities shown in device 100 is to provide a more effective treatment.

Referring again to **Figure 1B****,** fluid **120** is delivered from a fluid reservoir (not shown), which may be either part of the handle or in a separate reservoir, such as a plastic or glass tube serum, through the fluid delivery conduit **108** and out the fluid delivery tip **122** in the tip **104** of the device **100**. Fluid delivery may be used in the device for cleaning of the skin, as a vehicle for delivery of a therapeutic agent, or it may be the therapeutic agent itself, and/or the fluid may be an ionic agent to facilitate delivery of current 140 through the electrodes **130.** The fluid may include one or a plurality of suitable skin enhancing agents, and/or conductive ingredients, or other suitable agents for skin cleaning and skin enhancement or facilitation of current delivery, such as water, salts, ionic or non-ionic surfactants, preservatives, alcohol, glycerol, gel, and other similar agents. Various mixtures of these agents may be formulated into fluids with various conductivity levels, depending on the desired application. Preferably, at least one of the fluids used is a "highly conductive fluid" or a "fluid with a high conductivity" meaning a fluid with a conductivity from about 1,000 to about 100,000 (µSiemens/cm) to facilitate current delivery. Other fluids, such as a "fluid with a low conductivity", meaning a fluid with a conductivity from about 0.1 to about 999 (µSiemens/cm), are used in other applications, such as cleaning, and/or delivery of a skin enhancing or therapeutic agent. A highly conductive fluid is used to provide a conductive path through the skin. At least one fluid with a conductivity of at least 500 to about 50,000 uSiemens/cm is used.

Therapeutic or skin enhancing fluids useful in the device **100** may be of a variety of therapeutic agents. For example, the fluid may be a skin treatment liquid, a lotion liquid, and/or a vitamin liquid, or a combination thereof. The fluid may also be a pharmacologically-active agent, where the fluid carries a chemical agent of a suitable concentration. Examples of such agents include TCA (trichloroacetic acid), a glycolic acid including an alphahydroxy acid (AHA), a lactic acid, a citric acid, and phenol, alone or in combination with other agents or fluids. Examples of other therapeutic or skin enhancing agents include type A botulinum toxine, phosphatidylcoline, aminophylline, hyaluronic acid, L-carnitine, vitamins, amino acids, collagen, lidocaine, heparin, elastine, compounds for Mesotherapy procedures, glutathione, hormone replacement agents, hyaluronidase, MTE-4 (Copper-Manganese-Zinc Sulphate-Chromium), ionic skin tissue growth gels, enzymes, peptides and steroids.

Other ingredients can include plant and fruit derived ingredients, such as enzymes and stem cells derived from fruits and/or plants, etc. Since microdermabrasion is a controlled injury of the skin by abrading the surface layer to cause a wound healing response, other known healing and anti-inflammatory ingredients such as cortisone, aloe extract, etc. may be used to increase healing response time and also act as an anti-fungal, anti-viral, anti-bacterial and acaricidal activity against skin infections such as acne, etc. may be used individually or in any combination with other sterile fluids, drugs, and other skin enhancing and/or therapeutic agents.

Other agents and preferred viscosity parameters may be found in "Advanced drug delivery reviews", 56 (2004) 659-674.

Referring again to **Figure 1B****,** a vacuum **124** may be applied to the surface of the skin from a vacuum pump (not shown) through the vacuum conduit **112** and vacuum entry port **126** on the tip **104** of the device. Preferably, the vacuum pump which supplies the vacuum **124** to the device **100** has a rating of 2.9A, with a max flow rate of 2cu.ft/min, a power rating of 120 W, with a 60 Hz frequency, and preferably RoHS compliant, although other arrangements are possible. In general, the vacuum **124**, used during a treatment and applied to the surface (or just above) the skin of a patient, is a continuous flow and preferably can be adjusted with a flow control valve to increase or decrease vacuum pressure.

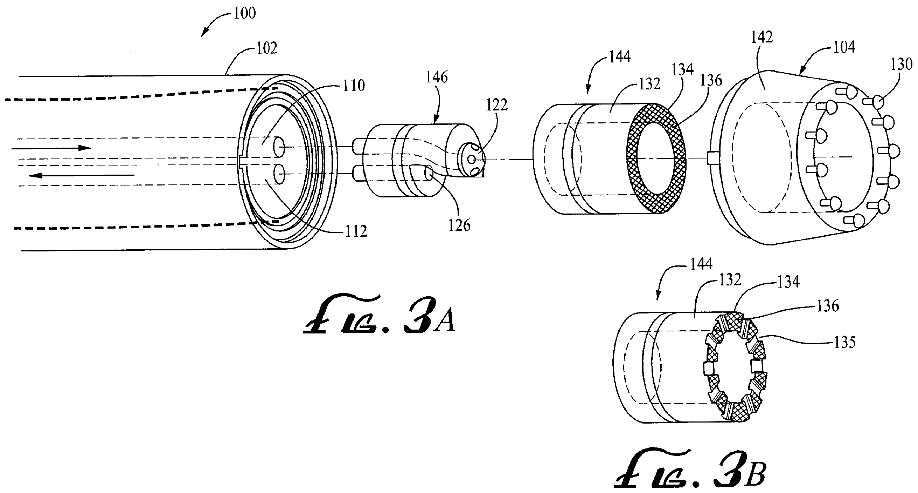

Referring now to Figure 3A, a skin abrading device **100**, having a plurality of removable, exchangeable, and attachable tips is shown. As shown in Figure 3A, the tip **104** of the device **100** comprises multiple nesting (e.g., interconnected) structures which are removable/attachable from the handle **102.** The outer structure **142** of the tip **104** comprises the electrodes **130** at the proximal end of the tip **104** and wiring (not shown) for delivering current 140 (not shown) to the electrodes **130.** Positioned within the outer structure **142,** is the intermediate structure **144,** which is also the abrading structure **132**. The inner structure **146** comprises the fluid delivery tip **122** and vacuum entry port **126.** When the structures **142, 144,** and **146** (i.e., tips) are assembled, the tip **104** of the device **100** will have the configuration shown in **Figures 1A, 1B****,** and **2A****-2C**.

That is to say, the inner structure **146** is located at the center of the tip. The outer structure **142** is located at the periphery of the tip. The intermediate structure **144** is located between the inner structure **146** and the outer structure **142**. The outer structure **142**, intermediate structure **144,** and the inner structure **146** are coaxial with each other and are in a ring shape. Preferably, the outer structure **142** and intermediate structure **144** form an outer ring and intermediate ring respectively at the tip. The outer ring and intermediate ring can be formed in a circular shape or a non-circular shape. Therefore, the abrading end portion forms at the intermediate ring and encircles the fluid delivery tip **122** and vacuum entry port **126** of the fluid delivery. The electrodes **130** are aligned at the outer ring to encircle the abrading end portion at the intermediate ring.

The outer structure **142,** intermediate structure **144** and inner structure **146** are connected to the handle **102** with a suitable connection, such as compression fitting, threaded fittings, etc. In an example, one or more of the outer structure **142**, intermediate structure **144,** and inner structure comprise stainless steel. In another example, the intermediate structure **144** comprises a reusable stainless steel abrading structure **132** having an abrading end portion **134** which has a diamond coated abrasive as the abrasive media **136.** In another example, the intermediate structure **144** comprises a disposable (preferably translucent) plastic abrading structure **132** having a disposable abrasive media **136** positioned on the abrading end portion **134**. In another example, the inner structure, comprising the fluid delivery tip **122** and the vacuum entry port **126,** are one or more of transparent, detachable, and/or disposable. Although the outer structure **142,** intermediate structure **144**, and inner structure **146** have been described herein as removable, exchangeable, and attachable, it will be understood by those of skill in the art that one or more of the outer structure **142**, intermediate structure **144,** and inner structure **146** maybe affixed to the handle **102** in a permanent, or not-easily removable fashion. However, in other examples, one or all of the structures **142-144** may be one piece in any arrangement or separate individual connections. For example, the device may comprise a handle **102** with an electric current node (i.e., electrode 130) in the middle surrounded by a fluid delivery piece **122** and an abrasive structure **132** making the outer edge of the handle. This is just an opposite arrangement from the arrangement shown in **Figure 3A****,** and as it will be understood by those of skill in the art, interrelationship of the various tips shown in the Figures is by way of example and other configurations are possible.

Referring now to **Figure 3B****,** another example, of the abrading structure **132** is shown. According to this example, the abrading end portion 134 of the abrading structure **132** comprises one or more grooves **135**. The grooves **135** may be differently shaped, such as rounded grooves, or slotted squares. The grooves **135** are provided to abrade the skin more effectively by stretching it, and to better guide skin debris into the vacuum. Preferably, to keep the vacuum **124** sealed, the grooves **135** are substantially even with the edge such that when the abrading structure **132** is applied to the skin, air does not escape. The grooves **135** may have a variety of thickness or radius, shape or design, for different skin types and applications, as will be understood by those of skill in the art. According to this example, extraction can be realized by pressing the abrading end portion **134** and grooves **135** to the skin, such that the grooves **135** act as a comedone extractor on a pore. For example, when the abrading end portion **134** having grooves **135** is pressed to the skin, oil and sebum will be released from the pores.

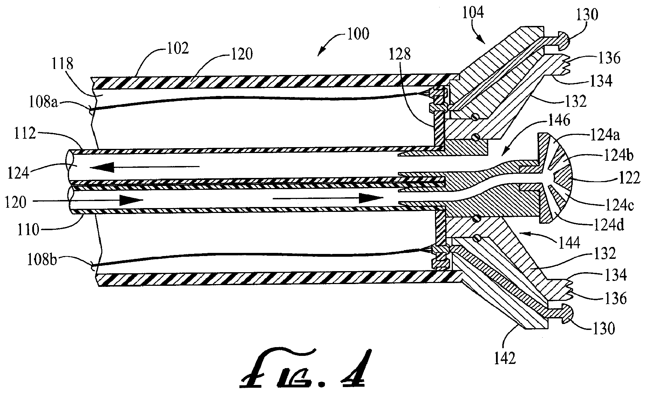

Referring now to **Figure 4****,** a partial side cut-away view of the device **100** having a wide-angle tip **104** is shown. As shown in Figure 4, the same numbers refer to the same features shown in **Figure 1B****,** with the differences noted below. According to this example, the tip **104** of the device is a wide angle tip, where the fluid **120** exits the tip **104** through a fluid delivery tip **122** having a plurality of apertures **124a - 124d.** According to this example, the wide angle tip allows for an increased area for fluid delivery and more apertures for fluid delivery. The interior **118** of the device **100** has one or more electrical conduits **108a, 108b,** which deliver current either to an electronics board **128**, which then delivers current to one or more electrodes **130**, or directly to the electrodes. As the tip **104** is a wide-angle tip, the electrodes are positioned further from the center of the tip **104** and in some examples, this allows for additional or wider electrodes **130** than the tapered tip **104** shown in **Figure 1A** and **Figure 1B****.** Positioned within the wide angle tip **104** is an abrading structure **132** having an abrading end portion **134**, which comprises an abrasive media **136.** Similarly to the electrodes **108,** the abrading end portion **124** and abrading media **132** are positioned further from the center of the device than the tapered tip **104** shown in **Figure 1A** and **Figure 1B****.** This example, may be used on a treatment area with a larger surface area that can accommodate the larger tip surface area. The various tips comprising the outer structure **142,** intermediate structure **144,** and inner structure **146,** shown in **Figure 4****,** may be removable, exchangeable, and attachable, and may be exchanged with other interchangeable tips **142-144,** of other dimensions, as described herein.

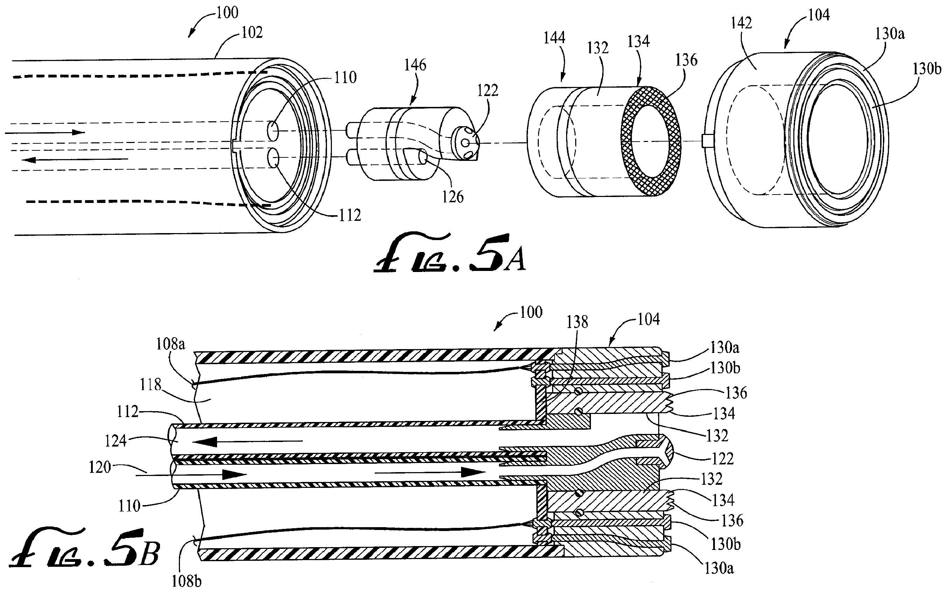

Referring now to **Figure 5A****,** a skin abrading device **100,** having a plurality of removable, exchangeable, and attachable tips, according to another example, is shown. Unless otherwise noted below, the same reference numbers refer to the same elements as described with reference to Figure 3. As shown in **Figure 5A****,** the tip **104** of the device **100** comprises multiple nesting (e.g., interconnected) structures which are removable/attachable from the handle **102.** As shown in **Figure 5A****,** the electrodes **130a** and **130b,** are concentric circles positioned within the outer structure **144** of the tip **104.** Positioned within the outer structure **144,** is the intermediate structure **144,** which is also the abrading structure **132.** The inner structure **146** comprises the fluid delivery tip **122** and vacuum entry port 126. Referring now to **Figure 5B****,** a partial side cut-away view of the device **100** shown in **Figure 5A****,** having electrodes **108a, 108b,** which are concentric circles is shown. When the structures **142, 144,** and **146** (i.e., tips) are assembled, the tip **104** of the device **100** will have the configuration shown in **Figure 5B****.** The outer structure **142,** intermediate structure **144** and inner structure **146** are connected to the handle **102** with a suitable connection, such as compression fitting, threaded fittings, etc. As shown in **Figure 5A** and **5B****,** the tip **104** is substantially linear with respect to the handle. However, in other examples, the tip **104** may be tapered as shown in **Figure 1A** or wide-angled, as shown in **Figure 4****.** The structures **142-146** may comprise any suitable metal such as stainless steel, or may be any suitable plastic that is transparent, detachable, and/or disposable, and may be removable, etc. as shown in Figure 5A, or substantially fixed, as described herein with respect to other examples, as will be understood by those of skill in the art.

Although the electrodes **130,** shown in Figure **5A** and other Figures, are shown as positioned on the outer structure **144**, the electrodes **130** may be positioned on the inner structure **146** and the fluid delivery portion **122** and/or the abrasive portion **132** may be positioned in the outer and intermediate structures **142** and **144**, in a variety of combinations, either removable/attachable or permanently part of the handle, as will be understood by those of skill in the art.)

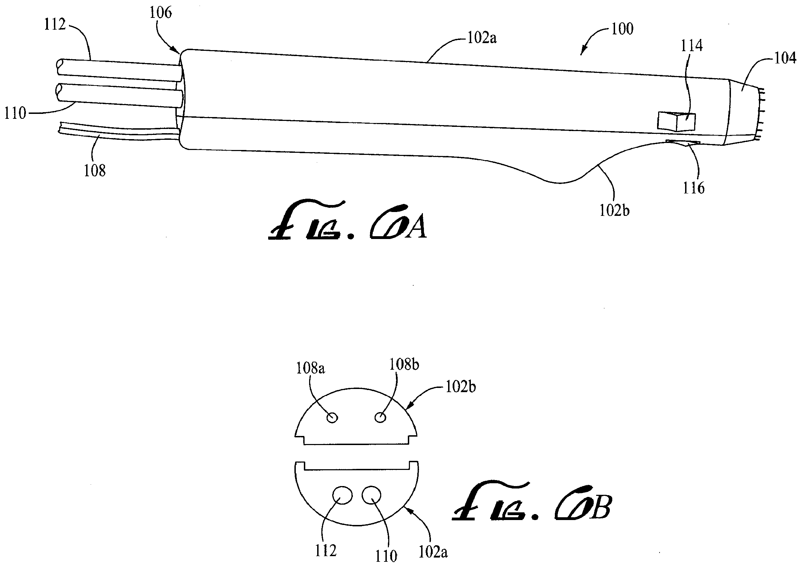

**Figure 6A** shows an alternate example of the skin abrading device **100.** As shown in **Figure 6A****,** the device **100** has a divided handle **102a** and **102b.** **Figure 6B** is a cross sectional view showing the divided handle **102a** and **102b** of **Figure 6A****.** As shown in **Figure 6B****,** the top portion of the handle **102a** comprises the fluid delivery conduit **110** and the vacuum conduit **112** and the bottom portion of the handle **102b** comprises the electrical conduits **108a**. The tip **104** of the device **100** shown in **Figure 6B****,** may have one or all of the configurations disclosed herein, including removable/interchangeable outer, intermediate and inner structures **142, 144** and **146** for the tip **104** portion of the device **100**, as shown in **Figures 3-5****.**

As shown in **Figures 1-6****,** each of the examples described comprises tip **104** having electrodes **130**, an abrading structure **132**, and fluid delivery **122.** However in other examples, the device may have only two of these features, such as the combination of electrodes **130** and fluid delivery **122**, without the electrode **130** feature, as will be understood by those of skill in the art.

According to another example, a method for treating a skin surface of a patient is provided, wherein one the above devices is employed to abrade the skin surface of a patient; deliver fluid to the surface of the skin; and apply current to the surface of the skin. These steps may be performed in the sequence described herein, or the sequence may be altered, depending on the type of procedures to be performed on the patient, as will be understood by those of skill in the art.

Firstly, the abrading end portion **134** of the abrading structure **132** of the device **100** is applied to the skin surface of a patient. Vacuum may optionally be applied to the skin surface to remove any residual debris, such as abrasive media and excess skin, either after or during the abrading portion of the treatment. Then, the skin surface is contacted with the abrading end portion **134** and abrasive media **136** of the device and the abrading end portion **134** of the device **100** is moved over the surface of the skin. Treatment can be substantially stationary in certain areas, or vary in the degree of motion, up to sweeping lines. Next, a fluid is provided to the skin surface through the fluid delivery tip **122** of the device **100.** Then, a current **140** is applied to the surface of the skin by transferring current from the electrodes **130** to the skin surface. The current 140 may be applied either to wet or dry skin.

Although the method is described above as being performed in a sequential manner, this is provided by way of example, and is only one of the possible protocols for the method of the invention. Accordingly, the various treatments, including skin abrasion, fluid delivery, and/or current delivery may be performed concurrently, or one at a time, in any order, depending on the patient needs and treatment given to any particular patient.

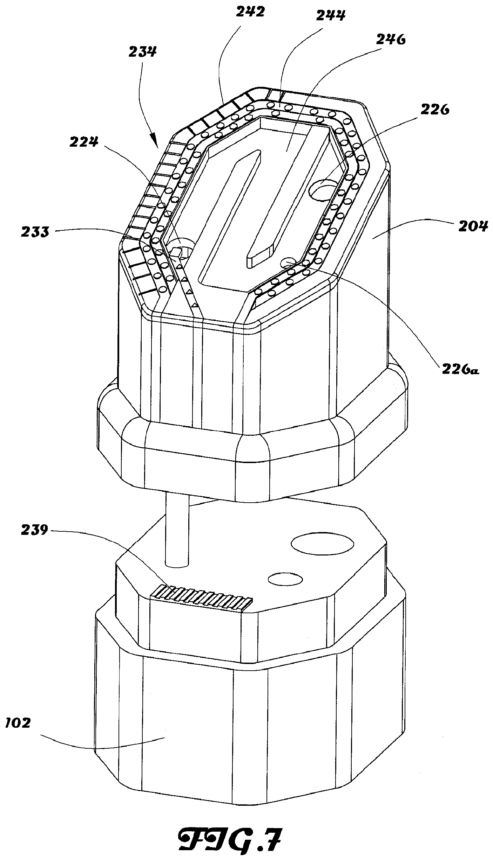

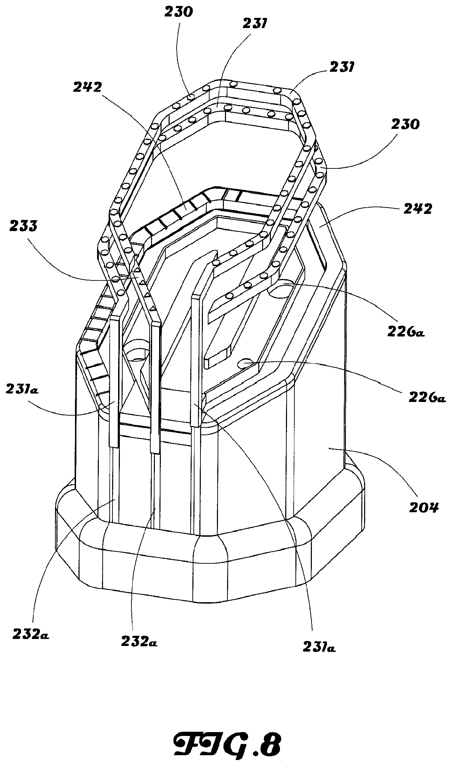

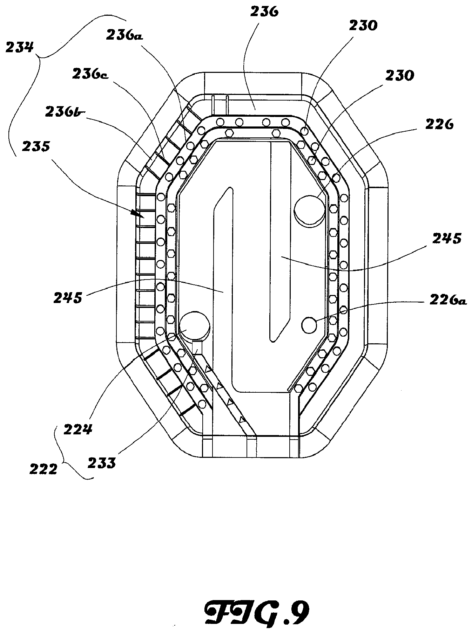

Another example, in **Figures 7 to 9** illustrates a modification of the tip **204** that detachably couples to the handle **102.** The tip **204** is a multi-functional tip to provide multiple functions. The tip **204** has a slanted skin applying surface, wherein the outer structure **242,** intermediate structure **244**, and the inner structure **246** are coaxial with each other and are formed at the slanted skin applying surface with respect to the handle **102**. The skin applying surface is a flat surface.

The outer structure **242** of the tip **204** comprises the abrading structure **232,** wherein the abrading end portion **234** of the abrading structure 232 comprises one or more abrading edges **236a, 236b.** The abrading structure forms an abrasive crown. In **Figure 7****,** the abrading structure **232** comprises an inner abrading edge **236a** and an outer abrading edge **236b**, wherein the inner abrading edge **236a** and outer abrading edge **236b** form in a ring shape, which can be a non-circular ring shape or a circular ring shape. The abrading end portion **234** of the abrading structure **232** comprises a plurality of connecting abrading edges **236c** spaced apart with each other and extended between the inner abrading edge **236a** and outer abrading edge **236b** to form a crown shaped abrading structure. A plurality of grooves **235** are formed between every two of the connecting abrading edges **236c.** The grooves **235** may be differently shaped, such as rounded grooves, or slotted squares. The grooves **135** are provided to abrade the skin more effectively by stretching it, and to better guide skin debris into the vacuum. Of course, the abrasive media **236** can also be replaceably placed at the abrading end portion **234** of the abrading structure **232** between the inner abrading edge **236a** and outer abrading edge **236b.**

The intermediate structure **244** comprises the electrodes **230** arranged in a ring shape. At least one electrode ring **231** is provided, wherein the electrodes **230** are spacedly formed at the electrode ring **231**. In **Figure 7****,** two electrode rings **231**, i.e. inner and outer electrode rings, are provided, wherein the outer electrode ring **231** is encircled within the inner abrading edge **236a** and the inner electrode ring **231** is encircled within the outer electrode ring **231**. Each electrode ring **231** can provide at least one of operations of electroporation, microcurrent, iontophoresis, sonophoresis, galvanic, ultrasound, ultrasonic cavitation, acoustic cavitation, mesotherapy, radio frequency, and/or hot and cold therapies. The two electrode rings **231** can provide two different operations respectively. Therefore, two different sets of electrodes **230** are provided at the inner and outer electrode rings **231** respectively. For example, one of the electrode rings **231** is to produce an electrical stimulant function, and another electrode ring **231** is to produce heat.

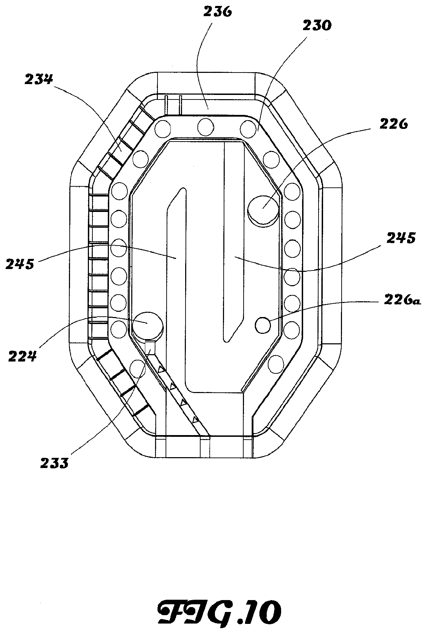

It would be acceptable that one single electrode ring **231** is replaceably formed at the intermediate structure **244** as shown in **Figure 10****.** The single electrode ring **231** can be a sonic brush tip in **Figure 10****.**

Each of the electrode rings **231** is replaceable, detachable, and/or disposable. Each electrode ring **231** has a latch **231a** extended from the electrode ring **231**, wherein the latch **231a** is slot-in the latch slot **232a** at the sidewall of the tip **204** to detachably couple the electrode ring **213** at the slanted skin applying surface of the tip **204.** The electrode rings **231** are attached to the removable tip and can provide multiple frequencies.

A terminal **239** is provided at the handle **102** and is electrically linked to the control circuit **138.** When the tip **204** couples to the handle **102,** the electrodes **230** at the intermediate structure 244 will electrically contact and connect with the terminal **239.**

The inner structure **246** comprises a fluid delivery structure, wherein the fluid delivery structure comprises the fluid delivery tip **222** and vacuum entry port **226**. The fluid delivery tip **222** has at least one aperture **224**, wherein the aperture **224** is formed at the slanted skin applying surface of the tip **204.** The vacuum entry port **226** is also formed at the slanted skin applying surface of the tip **204** and is located away from the aperture **224.**

The intermediate structure **244** comprises the fluid electrode terminal **233** extended toward the aperture **224** to electrify the fluid when the fluid is ejected right at the aperture **244.**

The inner structure **246** further comprises a plurality of fluid delivery walls **245** extended between the aperture **224** and the vacuum entry port **226** to form a fluid detouring path. When the fluid is ejected from the aperture **224,** the fluid is guided and detoured along the fluid detouring path to the vacuum entry port **226.** Therefore, the fluid detouring path will prolong the traveling distance of the fluid from the aperture **224** to the vacuum entry port **226.**

In **Figure 7****,** two fluid delivery walls **245** are extended from two opposite sides, i.e. first and second sides, of a boundary wall that partitions the inner structure **246** into two side sections and a mid-section. The boundary wall is the boundary of the inner structure **246**. Therefore, the boundary wall is the wall between the inner structure **246** and the intermediate structure **244.** One of the fluid delivery walls **245** is extended from the first side of the boundary wall toward the second side thereof to form a first cornering region. Another fluid delivery wall **245** is extended from the second side of the boundary wall toward the first side thereof to form a second cornering region. The aperture **224** and the vacuum entry port **226** are formed at the two side sections and are located at two ends of the fluid detouring path. Therefore, the fluid will travel from one side section to another side section through the mid-section, wherein the fluid will pass the first and second cornering regions. Preferably, the fluid delivery walls **245** are extended in parallel. Therefore, the fluid detouring path is a zigzag path that the fluid travels in a zigzag manner from the aperture **224** to the vacuum entry port **226.**

An additional vacuum entry port **226a** is provided at the fluid detouring path between the aperture **224** and the vacuum entry port **226.** The size of the additional vacuum entry port **226a** is smaller than the size of the vacuum entry port **226.** The additional vacuum entry port **226a** will pull a small amount of fluid first before the vacuum entry port **226** pulls the rest of fluid. Preferably, the additional vacuum entry port **226a** is located right after the second cornering region.

In an example, the outer structure **242**, intermediate structure **244,** and the inner structure **246** are integrated with the tip **204** at the skin applying surface. Only the electrode rings **231** are replaceably attached to the intermediate structure **244.** The abrasive media **236** is optionally placed at the outer structure **242.** Without the abrasive media **236,** the inner abrading edge **236a,** outer abrading edge **236b,** and connecting abrading edges **236c** at the outer structure **242** can perform the abrading operation.

The described device basically uses the electric current to stimulate blood circulation to increase the absorption of the liquid, similar to how the skin absorbs more when exercising or sweating from heat, the pores become more permeable. The electric currents to be used do cause the similar effect of softening the pores to allow liquid to penetrate deeper under the skin.

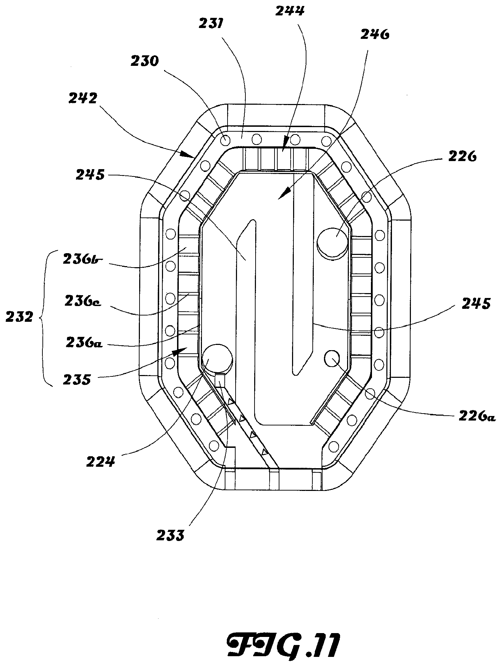

**Figure 11** shows the alternative of the tip **204** that has a slanted skin applying surface, wherein the inner structure **246,** including the aperture **224**, vacuum entry port **226** and fluid detouring path, remains the same. Only the outer structure **242** and intermediate structure **244** are interchanged. The electrode ring **231** is formed at the outer structure **242** and the abrading structure **232** is formed at the intermediate structure **244.**

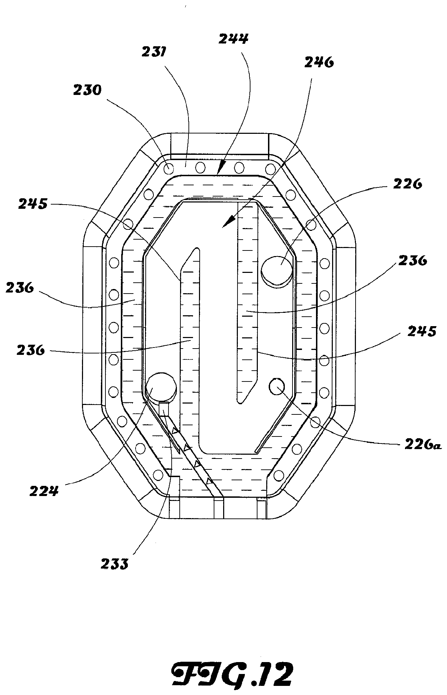

**Figure 12** shows another alternative of the tip **204.** The inner structure **246,** including the aperture **224,** vacuum entry port **226** and fluid detouring path, remains the same. The electrode ring **231** is formed at the outer structure **242.** The abrading structure **232** is formed at the intermediate structure **244.** The modification in **Figure 12** is that the abrasive media **236** is placed at the abrading end portion **234** and is placed at the top surfaces of fluid delivery walls **245** to increase the abrading surface of the tip **204.**

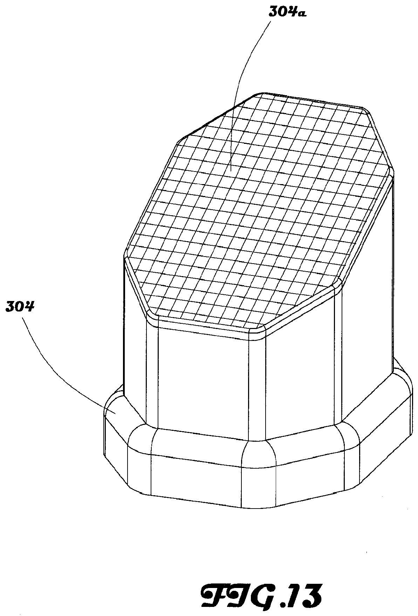

Another example, in **Figure 13** illustrates a modification of the tip 304 that detachably couples to the handle **102**. The tip **304** is an electrode skin treating tip which comprises an electrode film **304a** provided at the slanted skin applying surface for generating a specific electrical current such as of electroporation, microcurrent, iontophoresis, sonophoresis, galvanic, ultrasound, ultrasonic cavitation, acoustic cavitation, mesotherapy, radio frequency, and/or hot and cold therapies. The electrode film **304a** can also be a light film for generating a specific light wave for skin treatment. The electrode skin treating tip **304** can be attached to the handle **102** after the multi-functional tip **204** is removed. Therefore, the multi-functional tip **204** and the electrode skin treating tip **304** are interchangeable. It is worth mentioning that when the electrode skin treating tip **304** is used, the fluid delivery will not be turned off. Therefore, no aperture **224** and vacuum entry port **226** is formed at the electrode skin treating tip **304.**

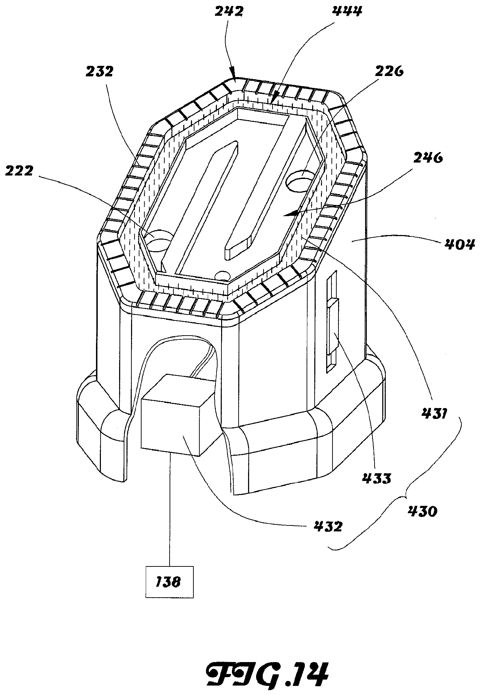

An example in **Figure 14** illustrates a further modification of the tip **404** that detachably couples to the handle **102.** The tip **404** is a micro-needle skin treating tip, which is also the multi-functional tip **204** to provide multiple functions. Similar to the multi-functional tip **204** in **Figure 7**, the micro-needle skin treating tip **404** has a slanted skin applying surface, wherein the outer structure **242,** intermediate structure **444,** and the inner structure **246** are coaxial with each other and are formed at the slanted skin applying surface. The outer structure **242** of the tip **404** comprises the abrading structure **232**. The inner structure **246** comprises the fluid delivery tip **222** and vacuum entry port **226**. The difference between the multi-functional tip **204** and the micro-needle skin treating tip **404** is that the intermediate structure **444** comprises a micro-needle assembly **430** having a plurality of micro-needles **431** provided at the skin applying surface between the outer structure **242** and the inner structure **246.**

The micro-needle assembly **430** is another example of a structure to penetrate fluid delivered through the skin that further comprises a vibrator **432** supported in the tip **404.** The vibrator **432** is connected to the control circuit **138** and is linked to the micro-needles **431.** During operation, the vibrator **432** will generate a vibration force to vibrate the micro-needles **431,** so that the micro-needles **431** will drive to reciprocatingly move and puncture into the skin surface. The vibrator **432** can also be a sonic vibrator to generate sonic wave to vibrate the micro-needles **431**. Therefore, the micro-needle skin treating tip **404** provides a micro-needling treatment for improving the skin complexion, wrinkle reduction and facial rejuvenation. The micro-needle skin treating tip **404** will repair skin damage from the sun, from acne, from injuries etc. By making tiny puncture wounds in the skin via the micro-needles **431**, causes a wound healing reaction that stimulates the skin to produce collagen to repair the controlled injury. The micro-needle assembly **430 f**urther comprises a needle leveling adjustor **433** provided at the sidewall of the tip **404,** wherein the level of depth of the micro-needles **431** can puncture the skin will be adjusted by the needle leveling adjustor **433**.

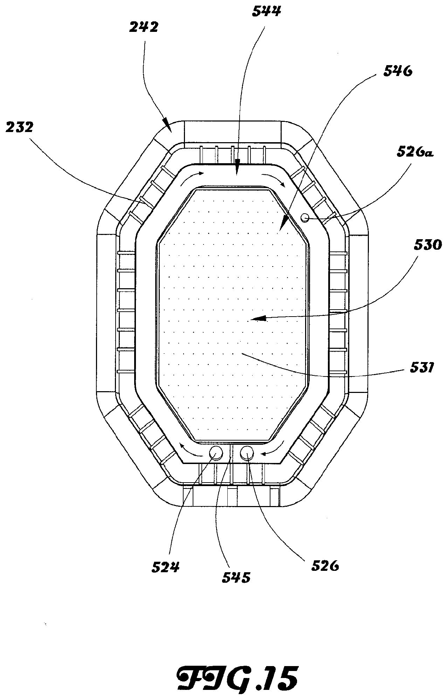

**Figure 15** shows another alternative example of the micro-needle skin treating tip **504.** The micro-needle skin treating tip **504** has a slanted skin applying surface, wherein the outer structure **242,** intermediate structure **544,** and the inner structure **546** are coaxial with each other and are formed at the slanted skin applying surface. The outer structure **242** of the tip **504** comprises the abrading structure **232.**

The intermediate structure **544** comprises the fluid delivery structure having an aperture **524,** a vacuum entry port **526,** and an additional vacuum entry port **526a.**

The inner structure **546** further comprises a fluid delivery wall **545** extended between the aperture **524** and the vacuum entry port **526** to form a fluid detouring path. When the fluid is ejected from the aperture **524,** the fluid is guided and detoured along the fluid detouring path to the vacuum entry port **526.** Therefore, the fluid detouring path will prolong the traveling distance of the fluid from the aperture **524** to the vacuum entry port **526.** The fluid delivery wall **545** is extended between two opposite sides of the boundary wall that partitions the intermediate structure **544** into a loop structure, wherein the aperture **524** and the vacuum entry port **526** are located at two ends of the fluid detouring path respectively, so that the fluid travels around the inner structure 546 from the aperture 524 to the vacuum entry port 526.

The inner structure 546 comprises a micro-needle assembly 530 having a plurality of micro-needles 531 provided at the skin applying surface within the inner structure 546. The vibrator 432 and the needle leveling adjustor 433 as disclosed in Figure 14 will also be employed in the micro-needle assembly 530. So, the vibrator 432 will generate a vibration force to vibrate the micro-needles 531, so that the micro-needles 531 will drive reciprocatingly to puncture into the skin surface. The level of the micro-needles 531 will be adjusted by the needle leveling adjustor 433 in order to adjust how deep the micro-needles 431 will to be punctured into the skin surface.

The multi-functional tip 204, the electrode skin treating tip 304, and the micro-needle skin treating tips 404, 504 are interchangeable. Figures 1-15 disclose examples of the present disclosure. Further, the embodiments of the present disclosure do not encompass the above examples and the method for transdermal fluid delivery.

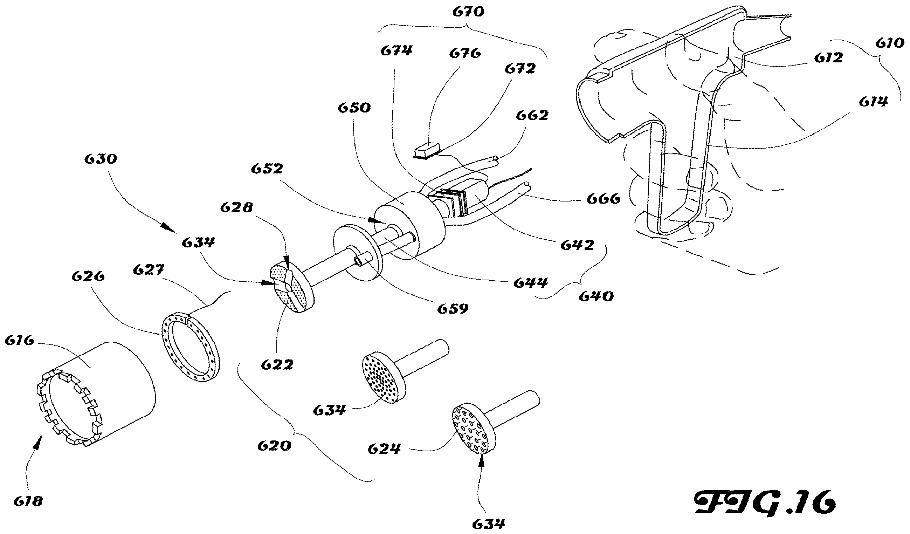

Figure 16 shows an embodiment of the apparatus of the invention. The apparatus for transdermal fluid delivery comprises a handle 610, a multi-functional tip 620, a fluid delivery structure 630, and a tip driver 640.

The handle 610 in this embodiment is an angled handle which comprises a casing 612 and a hand grip 614 inclined and extended from the casing 612. The casing 612, which is a hollow casing, has a front working end and a rear communicating end. The hand grip 614 is extended from the casing 612 between the working end and the communicating end, wherein an angle between the casing 612 and the hand grip 614 should be less than 90 degrees. The casing 612 comprises a detachable cap 616 detachably coupled at the casing 612, wherein the working end is defined at the detachable cap 616. The working end of the detachable cap 616 has a crown shaped outer edge 618 to apply pressure on the skin to perform pressure extractions.

The handle 610 is ergonomically designed, wherein the handle 610 can be held by a right or left-handed user. The casing 612 can be held by the thumb and the index finger of the user and the hand grip 614 can be held by the middle finger, ring finger, little finger and palm as illustrated in Figure 16. During operation of the device, the palm of the user may not be resting on a surface being treated. The angled handle **610** will give the fingers of the user more precise control of the working end of the casing **612** by the support of the palm of the user. That is to say, the palm support at the hand grip **614** will relieve the pressure at the fingers when held at the casing **612.**

The multi-functional tip **620** has a skin applying surface located at the working end of the handle **610,** wherein the skin applying surface is capable of contacting with a user skin. In **Figure 16****,** a plurality of abrading elements **622** are provided at the skin applying surface. In alternative mode, a micro-needle assembly **624** having a plurality of micro-needles is provided at the skin applying surface.

The multi-functional tip **620** further comprises an electrode module comprising a plurality of electrodes **626** encircled around the skin applying surface in a detachably mounting manner. The electrodes **626** are arranged in a ring configuration to surround the skin applying surface. The electrodes **626** are built-in with an inner side of the detachable cap **616** adjacent to the crown shaped outer edge **618** thereof. Therefore, the electrodes **626** can be replaced, detached, and/or disposed by the detachable engagement of the detachable cap **616.** That is to say, the electrodes **626** of the electrode module will be located around the abrading elements **622** and/or the micro-needle assembly **624** on the skin applying surface. A vacuum inlet **621** is formed between the electrodes **626** of the electrode module and the abrading elements **622**/the micro-needle assembly **624.**

The electrodes **626** will generate a desired function, such as iontophoresis, electroporation, ultrasound, or photomechanical wave. For electroporation, high voltage current is applied to the skin producing hydrophilic pores in the intercellular bilayers via momentary realignment of lipids. For phonophoresis, ultrasound pulses are passed through the probe into the skin fluidizing the lipid bilayers by the formation of bubbles caused by cavitation. For iontophoresis, a current passed between the active electrode and the indifferent electrode repelling drug away from the active electrode and into the skin. All the electrodes 626 can be configured to provide the same desired function. Or, each of the electrodes 626 can be configured to provide a particular function, so that the electrodes 626 will provide different functions at the same time when contacting with the skin. Therefore, different electrical frequencies are generated to stimulate different and wider range of cells types and skin depths from the surface to underneath so as to cause multiple reactions from the skin.

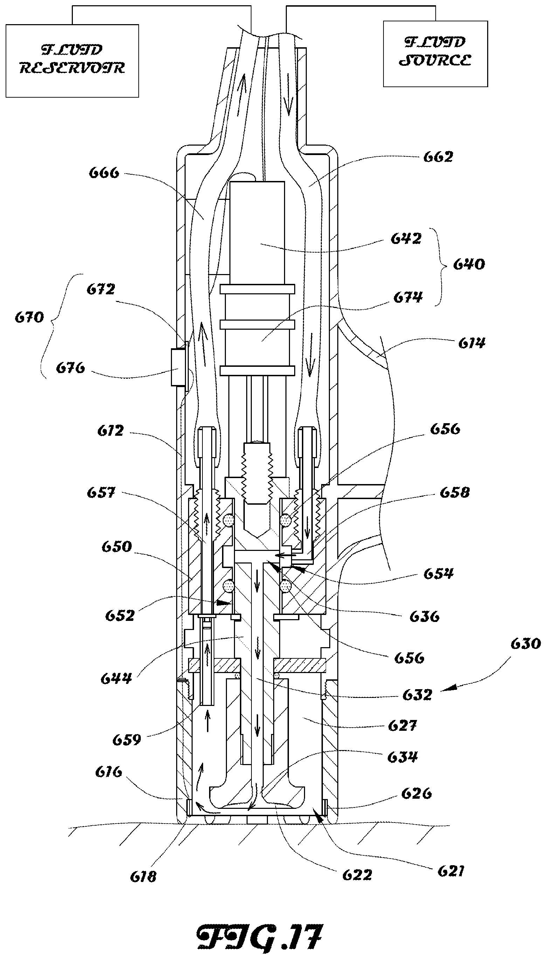

**Figures 16** and **17** show the tip driver **640** secured and supported in the casing **612** between the working end and the communicating end. The tip driver **640** comprises a driving unit **642** supported in the casing **612** and a driving shaft **644** operatively extended from the driving unit **642** to the multi-functional tip **620.** The driving unit **642** is operated to generate a movement at the skin applying surface of the multi-functional tip **620** via the driving shaft **644.** For example, with the abrading elements **622** on the skin applying surface, the driving unit **642** will drive the skin applying surface of the multi-functional tip **620** to rotate via the driving shaft **644.** It is preferred that the driving unit **642** will generate a reciprocating movement to rotate the multi-functional tip **620** back and forth. With the micro-needle assembly **624** on the skin applying surface, the driving unit **642** will drive the skin applying surface of the multi-functional tip **620** to slide within the casing **612** via the driving shaft **644.** It is preferred that the driving unit **642** will generate a reciprocating movement to move the multi-functional tip **620** front and back, which is aligned with a centerline of the casing **612**. During the operation of the device, the user will hold the handle **610** stably and stationary, and the skin applying surface of the multi-functional tip **620** is driven to move to contact with the user's skin.

The driving shaft **644** has at least a hollow portion extended to the multi-functional tip **620.** It is preferred that the driving shaft **644** is made of stainless steel.

**Figures 16** and **17** further show the fluid delivery structure **630** to guide a flow of fluid to the skin applying surface of the multi-functional tip **620**. The fluid delivery structure **630** has a fluid channel **632** defined at the hollow portion of the driving shaft **644** and at least an aperture **634** formed on the skin applying surface of the multi-functional tip to communicate with the fluid channel **632.** Therefore, the driving shaft **644** has a multifunction of driving the skin applying surface of the multi-functional tip to move and also while guiding the fluid through the fluid channel **632** to the skin applying surface of the multi-functional tip **620** at the aperture **634** at the same time.

The fluid will be directly ejected right on the skin applying surface of the multi-functional tip **620** at the aperture **634** when the skin applying surface of the multi-functional tip **620** is contacted with the user skin, so that the fluid delivery structure **630** of the invention is the most optimal way to transdermally penetrate fluid deeper in the skin.

In **Figure 16**, the aperture **634** is located at the center of the skin applying surface of the multi-functional tip **620,** wherein the abrading elements **622** are radially formed at the skin applying surface. A plurality of fluid distributing channels **628** are radially and outwardly extended from the aperture **634** to the electrodes **626**. Each of the fluid distributing channels **628** is formed at a gap between two adjacent abrading elements **622.** Therefore, the fluid will be evenly distributed on the skin applying surface through the fluid distributing channels **628** and toward the electrodes **626.** It is realized that the apertures **634** can also be located in other areas other than the center of the skin applying surface within the abrading elements **622,** such as the sides as well.

In **Figure 16****,** two or more of apertures **634** can be provided at the skin applying surface to deliver the fluid to the micro-needle assembly **624**. Two or more of apertures **634** can also be provided at the skin applying surface and can serve as a jet propulsion outlet to deliver the fluid in a high rate of speed.

In **Figure 16****,** the multi-functional tip **620** is detachably coupled at the free end of the driving shaft **644.** When the multi-functional tip **620** is detachably coupled at the free end of the driving shaft **644,** the aperture **634** is communicatively linked to the fluid channel **632**. Therefore, different types of multi-functional tip **620** are interchangeable and coupled at the driving shaft **644.** In this embodiment, three different types of multi-functional tip **620** are provided, i.e. the multi-functional tip **620** with the abrading elements **622,** the multi-functional tip **620** with the micro-needle assembly **624,** and the multi-functional tip **620** with the jet propulsion outlet. All these multi-functional tips **620** can be detachably coupled at the driving shaft **644** to guide the fluid to be ejected at the skin applying surface.

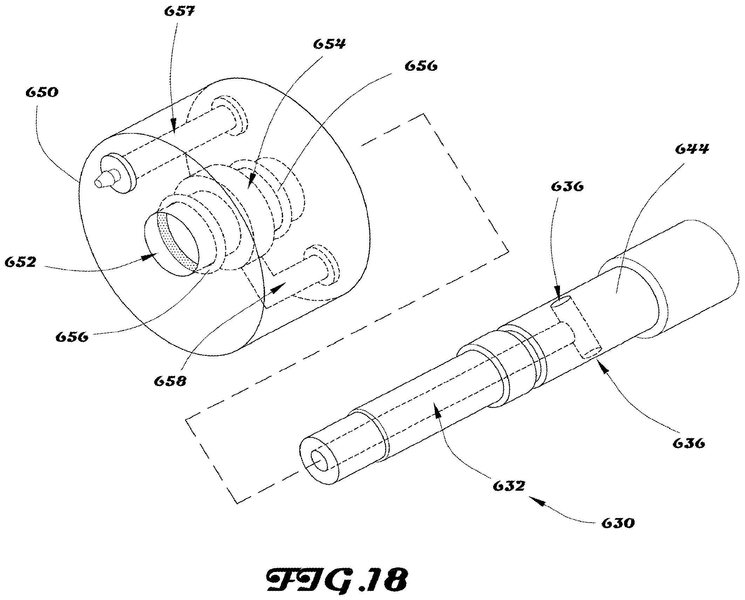

In **Figures 17** and **18****,** the fluid delivery structure **630** further has at least a fluid inlet **636** transversely formed at the driving shaft **644** to guide the fluid entering into the fluid channel **632** from the fluid inlet **636** and to guide the fluid exiting toward the aperture **634**. It is preferred that two fluid inlets **636** are formed at the driving shaft 644 are perpendicular to the fluid channel 632.

In **Figures 16** and **17** the device further comprises a support member **650** secured and supported in the casing **612** in a non-movable manner. The support member **650** can be removably mounted in the casing **612** to support the driving unit **642**. The support member **650** has a through center slot **652**, wherein the driving shaft **644** is supported by and extended through the center slot **652** of the support member **650**. In this embodiment, the driving shaft **644** is movable and the support member **650 i**s stationary. During the operation of the driving unit **642,** the driving shaft **644** will be moved and vibrated at any direction. The support member **650** will restrict the movement of the driving shaft **644** in only one direction. For example, the support member **650** will ensure the driving shaft **644** to be rotated within the center slot **652** or to be slid back and forth within the center slot **652**. So, the support member **650** will prevent any unwanted vibration of the driving shaft **644**. The support member **650** also supports the driving shaft **644** in the casing **612** because the driving shaft **644** must be long enough to extend from the driving unit **642** to the multi-functional tip **620** within the casing **612**. Therefore, the support member **650** is located between the driving unit **642** and the multi-functional tip **620,** wherein the rear side of the support member **650** faces toward the driving unit **642** and the front side of the support member **650** faces toward the multi-functional tip **620.**

In **Figures 17** and **18****,** the fluid inlet **636** at the driving shaft **644** is located within the support member **650**. To guide the fluid into the fluid inlet **636** through the support member **650,** the support member **650** has an interior fluid cavity **654** for delivering the fluid from a fluid source to the interior fluid cavity **654**. Then, the fluid in the interior fluid cavity **654** will enter into the fluid channel **632** from the fluid inlet **636.** The interior fluid cavity **654** is radially projected from the center slot **652** of the support member **650**, so that when the driving shaft **644** is extended through the center slot **653**, the fluid inlet **636** can communicate with the interior fluid cavity **654**.

As the driving shaft **644** is movable and extended through the center slot **652** of the support member **650** to locate the fluid inlet **636** within the interior fluid cavity **654** of the support member **650**, the fluid is able to enter into the fluid inlet **636** from the interior fluid cavity **654** when the driving shaft **644** is moved with respect to the support member **650.**

The size of the interior fluid cavity **654** is configured in response to the movement of the driving shaft **644.** When the driving shaft **644** is rotated within the center slot **652,** the width of the interior fluid cavity **654** should be larger than a diameter of the fluid inlet **636**. When the driving shaft **644** is slid within the center slot **652,** the width of the interior fluid cavity **654** should be larger than a traveling displacement of the fluid inlet **636.**

Two sealing elements **656** are embedded at an inner wall of the center slot **652** to fluidly seal the fluid inlet **636** within the interior fluid cavity **654** and between the two sealing elements **656.** The sealing elements **656** are two sealing rings embedded in the inner wall of the center slot **652** to engage with the driving shaft **644,** wherein the driving shaft **644** is still movable when the sealing elements **656** are engaged with the driving shaft **644**. The sealing element **656** will only seal the fluid within the interior fluid cavity **654** to prevent the leakage of the fluid within the center sot **652** when the driving shaft **644** is moved.

In **Figures 16****,****17****,** and **18****,** the support member **650** further has a fluid guiding passage **658** extended from the rear side of the support member **650** to the interior fluid cavity **654,** wherein the fluid is guided to flow from the fluid guiding passage **658** to the interior fluid cavity **654** before it is guided to flow to the fluid channel **632** from the fluid inlet **636.** The fluid guiding passage **658** is an elongated passage. An inlet of the fluid guiding passage **658** is formed at the rear side of the support member **650** and an outlet of the fluid guiding passage **658** is formed at the interior fluid cavity **654**. A first fluid tube **662** is extended from the inlet of the fluid guiding passage **658** and is extended out of the communicating end of the casing **612** to operatively link to the fluid source.

The fluid delivering path of the fluid from the fluid source to the skin applying surface of the multi-functional tip **620** is described as follows. The fluid is stored in the fluid source and is guided to flow from the fluid source to the fluid guiding passage **658** by the first fluid tube **662.** The fluid source may generate an optional pumping force to pump the fluid to the fluid guiding passage **658.** The fluid is then guided into the interior fluid cavity **654** by the fluid guiding passage **658.** The fluid will enter into the fluid channel **632** from the fluid inlet **636**. Finally, the fluid will be delivered right on the skin applying surface of the multi-functional tip **620** at the aperture **634.** Without the pumping force generated by the fluid source, the fluid is pulled from the fluid source, through the fluid guiding passage **658,** the interior fluid cavity **654,** to delivered right on the skin applying surface of the multi-functional tip **620** at the aperture **634** by the vacuum source.

The support member **650** further has a vacuum passage **657** extended through the support member **650** and a vacuum port **659** for vacuuming the fluid after the fluid is delivered to the skin applying surface of the multi-functional tip **620**. An inlet of the vacuum passage **657** is formed at the front side of the support member **650** and an outlet of the vacuum passage **657** is formed at the rear side of the support member **650**. It is preferred that the vacuum port **659** is extended from the inlet of the vacuum passage **657** toward the vacuum inlet **621** around the skin applying surface of the multi-functional tip **620**. A second fluid tube **666** is extended from the outlet of the vacuum passage **657** and is extended out of the communicating end of the casing **612** to operatively link to a fluid reservoir.

The fluid returning path of the fluid from the skin applying surface of the multi-functional tip **620** to the fluid reservoir is described as follows. The used fluid at the working end of the casing **612** is collected at the vacuum inlet **621** by the vacuum port **659** and is transmitted to the vacuum passage **657**. Then, the used fluid will be delivered to a fluid reservoir by the second fluid tube **666**. The fluid reservoir will generate a vacuum force to create a vacuum effect at the vacuum port **659**. When the fluid is delivered at the skin applying surface of the multi-functional tip **620,** the fluid will be electrified and conducted with the electrodes **626.**

In **Figure 17**, the center slot **652**, the fluid guiding passage **658,** and the vacuum passage **657** are parallel with each other at the support member **650.** It is important that the fluid will pass the electrodes **626** from the skin applying surface before the fluid is pulled back at the vacuum inlet **621**, so that the fluid will be electrified and conducted with the electrodes **626** to enable the liquid to penetrate into the skin longer and deeper.

A control module **670** is provided to control the operations of the electrodes **626** and the tip driver **640.** The control module **670** comprises a control circuit **672** operatively connected to the electrodes **626** and the tip driver **640**, and a transmission unit **674,** such as a gear box, operatively connected to the driving unit **642** to adjust amplitude of the driving shaft **644.** For example, the output of the rotational speed (rpm) of the driving unit **642** can be adjusted by the transmission unit **674,** so that the user is able to adjust the rotational speed of the multi-functional tip **620** via one or more control switches **676** provided on the handle **620.** The control switches **676** can also control and select the electrical frequencies of the electrodes **626**. An insulated wiring **627** is provided to connect the electrodes **626** with the control module **670** and is embedded in the detachable cap **616** to prevent the electric shock when the fluid is pulled back from the vacuum inlet **621.** A terminal is provided at the rear end of the detachable cap **616** to connect with the insulated wiring **627**, so that when the detachably cap **616** is detachably coupled at the casing **612**, the electrodes **626** are electrically connected to the control module **670**. It would be acceptable that the electrodes **626** are replaceable, detachable, and/or disposable by interchanging different detachable caps **616.** The control module **670** can also be located on the main unit (not shown) controlled by manual push button switches or controlled by a touch screen monitor that is connected to the handle **610.**

An alternative of the device, not covered by the present invention, can be formed without the driving unit, wherein the multi-functional tip **620** is driven to move by the flow of the fluid via the fluid delivery structure **630.** For example, when the fluid is guided to flow at the fluid channel **632** in a vortex manner to drive the multi-functional tip **620** to rotate. Or the apertures **634** on the skin applying surface have an ejecting angle, so that during the ejection of fluid at the apertures **634**, the multi-functional tip **620** is propelled to rotate.

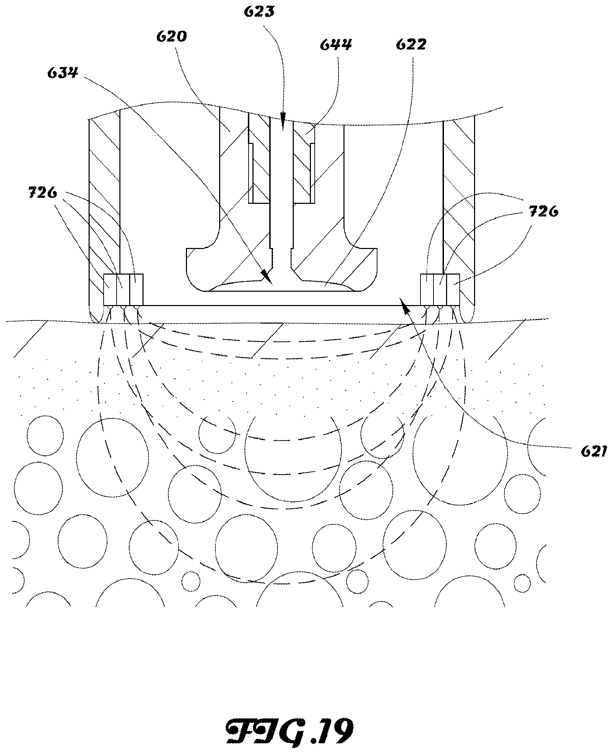

**Figure 19** shows a modification of the electrode module which comprises two or more different electrodes **726**. In **Figure 19****,** three different electrodes **726** are utilized and are configured into an inner electrode ring, an intermediate electrode ring, and an outer electrode ring which are coaxial with the skin applying surface of the multi-functional tip **620** orderly. The three electrode rings will generate different electrical frequencies to stimulate different and wider range of cells types and skin depths from the surface to underneath so as to cause multiple reactions from the skin. That is to say, the three different electrodes **726** can improve the skin structure affecting multiple layers of the skin, such as, epidermis, dermis, and hypodermis. It is worth mentioning that the fluid will guide to pass through different electrodes **726** from the abrading elements **622** on the skin applying surface to the vacuum inlet **621** at the perimeter of the outer electrode ring.

The apparatus of the present invention is an innovative apparatus of treating the skin to transdermally penetrate fluid deeper into the skin by means of simultaneous (1) abrasive peeling via the abrading elements **622,** (2) electrical stimulation via the electrodes **626**, (3) liquid infusion via the fluid delivered onto the skin applying surface, in order to improve the skin structure affecting multiple layers of the skin, such as, epidermis, dermis, and hypodermis.

More importantly, the fluid will be delivered right on the skin applying surface to evenly distribute on the abrading elements **622** and then to electrify with the electrodes **626** before the fluid is vacuumed back through at the vacuum inlet **621**. The traveling path of the fluid will be prolonged between the aperture **634** and the vacuum inlet **621** to ensure the fluid to pass through the abrading element **622** and the electrodes **626.** The skin abrading operation is automatic by the movement of the multi-functional tip **620.** Therefore, the apparatus of the present invention produces a new singular treatment to include three different skin treatment methods in one single device. That is to say, the user can simply hold the handle 610 stationary and place the skin applying surface of the multi-functional tip **620** on the skin surface with three different functions operating in conjunction with each other.

Therapeutically, when the interaction between the fluid and electrodes **626** occurs, it delivers a medicine in the fluid through the skin surface. It is a non-invasive method to enhance the effects on skin permeation and to enhance the absorption of medicine across the skin surface. It drives a charged substance, such as medication or a bioactive agent, transdermally by repulsive electromotive force, through the skin surface.

The three different skin treatment methods are interlinked with each other and are not independent functions from each other. That is to say, the three different skin treatment methods enhance each other's functions as a whole. The fluid will be delivered right on the skin applying surface to directly contact with the skin surface for liquid infusion. The fluid will also be flush to the skin surface when the abrading elements **622** are applied on the skin surface. The fluid will also interact with the electrodes **626** for electrical stimulation on the skin surface. It is worth mentioning that due to the vacuum effect at the vacuum inlet **621**, the fluid will be forced to vacuum at the vacuum inlet **621** from the apertures **634** to ensure the fluid to pass through the abrading elements **622** and the electrodes **626** before the fluid is pulled back at the vacuum inlet **621.**

The method, which does not form part of the present invention, for transdermal fluid delivery comprises the steps as follows:
(A) Hold the handle 610 stationary to locate the working end of the handle **610** on a skin surface.
(B) Deliver the fluid onto the skin applying surface of the multi-functional tip **620** at the aperture **634** which is formed on the skin applying surface. Therefore, the fluid can be directly delivered to the skin applying surface to contact with the skin surface. More particularly, the fluid is guided to pass through the hollow portion of the driving shaft **644** which serves as the fluid channel **632** to guide the fluid to the aperture **634** through the fluid channel **632.**
(C) Evenly distribute the fluid on the abrading elements **622** which are provided on the skin applying surface. The fluid can be evenly distributed on the abrading elements **622** through the fluid distributing channels **628.** Or, two or more apertures **634** are formed on the skin applying surface to evenly distribute on the abrading elements **622**.
(D) Drive the skin applying surface of the multi-functional tip **620** to move for abrasive peeling while the handle **610** is stationary. Without moving the handle **610**, the skin applying surface of the multi-functional tip **620** is driven to move by the tip driver **640**.
(E) Guide the fluid to be interacted with the electrodes **626** of the electrode module encircled around the skin applying surface. After the fluid interacting with the abrading elements 622, the fluid will pass to the electrodes 626 for electrical stimulation on the skin surface.
(F) Vacuum back the fluid at the vacuum inlet 621 formed at the perimeter of the electrode module to ensure the fluid to interact the abrading elements 622 and the electrodes 626 before the fluid is pulled back at the vacuum inlet 621. Due to the vacuum effect, the fluid will be forced to pass through the abrading elements 622 and the electrodes 626.

Although the present invention has been discussed in considerable detail with reference to certain preferred embodiments, other embodiments are possible. Therefore, the scope of the present invention is solely limited by the appended claims.

## Claims

1. An apparatus for transdermal fluid delivery said device having a handle structure (610), a multifunctional tip (620) with a skin applying surface, a fluid delivery structure (630), and a tip driver (640), the apparatus comprising:
a casing (612) within said handle structure (610) with a front working end and a rear communication end;
said handle structure (610) further comprising a hand grip (614) inclined and extended between said front working end and said rear communication end of said casing (612);
a detachable cap (616) coupled to said casing (612) at said working end of said casing (612);
a crown shaped outer edge (618) at a working end of said detachable cap (616) to apply pressure on a skin surface to perform pressure extractions;
said multifunctional tip (620) with said skin applying surface located within said detachable cap (616) at said working end of said casing (612) within said handle structure (610);
wherein said tip driver (640) comprises, a driving unit (642) and a driving shaft (644);
a support member (650) within said casing (612) located between said driving unit (642) and said multifunctional tip (620) to support said driving shaft (644) of said tip driver (640);
said support member (650) having a fluid guiding passage (658) extending from a rear side of the support member (650) to an interior fluid cavity (654), and that a first fluid tube (662) is extended from the inlet of the fluid guiding passage (658) and is extended out of the communicating end of the casing (612) to operatively link to the fluid source;
said support member (650) further having a vacuum passage (657) extended through the support member (650) and a vacuum port (659), and a second fluid tube (666) that is extended from the outlet of the vacuum passage (657) and is extended out of the communicating end of the casing (612) to operatively link to a fluid reservoir;
said support member (650) having a through center slot (652) wherein said driving shaft (644) of said tip driver (640) is operatively extended from said driving unit (642) to the multifunctional tip (620);
wherein said skin applying surface of said multifunctional tip (620) comprises a plurality of abrading elements (622) and an electrode module comprising a plurality of electrodes (626) encircling said abrading elements (622), with said electrode module attached to an inner side of said detachable cap (616);
wherein said support member (650), said first fluid tube (662), said second fluid tube (666), and said tip driver (640) are secured inside said casing (612) within said handle structure (610);
wherein said driving shaft (644) is operatively extended from said driving unit (642) to said multi-functional tip (620) through said through center slot (652) of said support member (650), wherein said driving shaft (644) is movable and said support member (650) is stationary;
wherein said multifunctional tip (620) comprising said plurality of abrading elements (622) and said plurality of electrodes (626) on said electrode module is detachably coupled at a free end of said driving shaft (644);
wherein said driving shaft (644) has at least a hollow portion extended to said multi-functional tip, wherein a fluid delivery channel (632) of said fluid delivery structure (630) is defined at said hollow portion of said driving shaft (644);
wherein said fluid delivery structure (630) further has a fluid inlet (636) transversely formed at said driving shaft (644) to guide a fluid entering into said fluid delivery channel (632) from the interior fluid cavity (654) and with said interior fluid cavity (654) located within said support member (650);
wherein said fluid delivery channel (632) has at least an aperture (634) located at an axial center of said skin applying surface of said multi-functional tip (620) to communicate with said fluid delivery channel (632) defined at said hollow portion of said driving shaft (644) and deliver fluid to the skin surface;
wherein said driving shaft (644) has a dual function of driving said skin applying surface of said multi-functional tip (620) to rotate while concomitantly guiding a flow of a fluid through said fluid delivery channel (632) to said skin applying surface of said multi-functional tip (620) at said aperture (634);
wherein said second fluid tube (666) comprises a vacuum inlet (621), wherein said vacuum passage (657) is extended through said support member (650) to transport a used fluid collected by said vacuum inlet (621) from said multifunctional tip by said vacuum port (659) to the fluid reservoir at a rear end of said support member (650); and
a control module (670) having a control circuit (672) operatively connected to the electrodes (626) to control the operations of the electrodes (626) and the tip driver (640) wherein a transmission unit (674) is operatively connected to said driving unit (642) of said tip driver (640) to adjust the amplitude of said driving shaft (644) of said tip driver (640).

2. The apparatus in claim 1 wherein said support member (650) is stationary and restricts a movement of said driving shaft (644) within said center slot (652) of said support member (650) only in one direction or back and forth within said center slot (650) of said support member (650) thereby preventing any unwanted vibrations of said driving shaft (644) when it is in operation.

3. The apparatus in claim 1 wherein said driving shaft (644) is movable and extended through said center slot of said support member (650) to locate said fluid inlet (636) transversely formed at said driving shaft (644) so that when said driving shaft (644) is moved with respect to said support member (650) a fluid is able to enter into said fluid inlet (636) from said fluid delivery channel (658).

4. The apparatus in claim 1 wherein said support member (650) further comprises two sealing elements (656) embedded at an inner wall of said center slot (652) of said support member (650) to fluidly seal said fluid inlet (636) within said interior fluid cavity (654) between said two sealing elements (656) to prevent leakage of said fluid within said center slot (652) of said support member (650) when the driving shaft (644) moves.

5. The apparatus in claim 1 wherein said fluid guiding passage (658) extends from the rear side of said support member (650) to said interior fluid cavity (654), wherein said fluid is guided to flow from said fluid guiding passage (658) to said interior fluid cavity (654) before it is guided to flow into said fluid delivery channel (632) from said fluid inlet (636).

6. The apparatus in claim 1 wherein said aperture (634) formed at said axial center of said skin applying surface defines a plurality of fluid distributing channels (628) radially and outwardly extended from said aperture (634) to said electrodes (626) wherein each of said fluid distributing channels (628) is formed at a gap between two adjacent abrading elements (622).

7. The apparatus in claim 1 wherein said multi-functional tip further comprises a micro-needle assembly having a plurality of micro-needles provided at said skin applying surface.

8. The apparatus in claim 1 wherein said fluid delivery channel (632) defined between said aperture (634) located at said axial center of said skin applying surface of said multi-functional tip and said vacuum inlet (621) ensures a flow of fluid to be delivered onto said skin applying surface through said aperture (634) and to interact with said abrading elements (622) and said electrodes (626) before said fluid is pulled back through said vacuum inlet (621) through said vacuum port (659), so that three different functions of skin treatments, that of, abrasive peeling, electrical stimulation, and liquid infusion are achieved in one step.

## Patentansprüche

1. Vorrichtung zur transdermalen Flüssigkeitsabgabe, wobei die Vorrichtung eine Griffstruktur (610), eine multifunktionale Spitze (620) mit einer Hautaufbringungsoberfläche, eine Fluidabgabeeinrichtung (630) und einen Spitzenantrieb (640), wobei die Vorrichtung umfasst:
ein Gehäuse (612) innerhalb der Griffstruktur (610) mit einem vorderen Arbeitsende und einem hinteren Anschlussende;
wobei die Griffstruktur (610) ferner einen Handgriff (614) umfasst, der geneigt ist und sich zwischen dem vorderen Arbeitsende und dem hinteren Anschlussende des Gehäuses (612) erstreckt;
eine abnehmbare Kappe (616), die an dem Arbeitsende des Gehäuses (612) mit dem Gehäuse (612) verbunden ist;
eine kronenförmige Außenkante (618) an einem Arbeitsende der abnehmbaren Kappe (616), um Druck auf eine Hautoberfläche auszuüben, um Druckextraktionen durchzuführen;
wobei die multifunktionale Spitze (620) mit der Hautaufbringungsfläche innerhalb der abnehmbaren Kappe (616) an dem Arbeitsende des Gehäuses (612) innerhalb der Griffstruktur (610) angeordnet ist;
wobei der Spitzenantrieb (640) umfasst: eine Antriebseinheit (642) und eine Antriebswelle (644);
ein Trägerelement (650) innerhalb des Gehäuses (612), das zwischen der Antriebseinheit (642) und der multifunktionalen Spitze (620) angeordnet ist, um die Antriebswelle (644) des Spitzenantriebs (640) zu stützen;
das Trägerelement (650), das einen fluidführenden Durchlass (658) aufweist, der sich von einer Rückseite des Trägerelements (650) zu einem inneren Fluidhohlraum (654) erstreckt, und in dem sich ein erstes Fluidrohr (662) vom Einlass des fluidführenden Durchlasses (658) aus bis zum Anschlussende des Gehäuses (612) erstreckt, um eine Wirkverbindung mit der Fluidquelle herzustellen;
wobei das Trägerelement (650) ferner einen Vakuumdurchgang (657), der sich durch das Trägerelement (650) erstreckt, und einen Vakuumanschluss (659) sowie ein zweites Fluidrohr (666) aufweist, das sich vom Auslass des Vakuumdurchgangs (657) erstreckt und aus dem Anschlussende des Gehäuses (612) herausragt, um eine Wirkverbindung mit einem Fluidreservoir herzustellen;
wobei das Trägerelement (650) eine durchgehende Mittelnut (652) aufweist, in dem sich die Antriebswelle (644) des Spitzenantriebs (640) operativ von der Antriebseinheit (642) zu der multifunktionalen Spitze (620) erstreckt;
wobei die Hautaufbringungsfläche der multifunktionalen Spitze (620) eine Vielzahl von Abriebelementen (622) und ein Elektrodenmodul mit einer Vielzahl von Elektroden (626) umfasst, die die Abriebelemente (622) angeordnet sind,
wobei das Elektrodenmodul an einer Innenseite der abnehmbaren Kappe (616) angebracht ist;
wobei das Trägerelement (650), das erste Fluidrohr (662), das zweite Fluidrohr (666) und der Spitzenantrieb (640) innerhalb des Gehäuses (612) innerhalb der Griffstruktur (610) befestigt sind;
wobei die Antriebswelle (644) sich operativ von der Antriebseinheit (642) zu der multifunktionalen Spitze (620) durch die durchgehende Mittelnut (652) des Trägerelements (650) erstreckt, wobei die Antriebswelle (644) beweglich und das Trägerelement (650) stationär ist;
wobei die multifunktionale Spitze (620), die die Vielzahl von Abriebelementen (622) und die Vielzahl von Elektroden (626) auf dem Elektrodenmodul umfasst, an einem freien Ende der Antriebswelle (644) abnehmbar gekoppelt ist
wobei die Antriebswelle (644) mindestens einen hohlen Abschnitt aufweist, der sich zu der multifunktionalen Spitze erstreckt, wobei ein Fluidzufuhrkanal (632) der Fluidabgabeeinrichtung (630) an dem hohlen Abschnitt der Antriebswelle (644) definiert ist;
wobei die Fluidabgabeeinrichtung (630) ferner einen Fluideinlass (636) aufweist, der quer an der Antriebswelle (644) ausgebildet ist, um ein in den Fluidabgabekanal (632) eintretendes Fluid aus dem inneren Fluidhohlraum (654) zu leiten, und wobei der innere Fluidhohlraum (654) innerhalb des Trägerelements (650) angeordnet ist;
wobei der Fluidabgabekanal (632) mindestens eine Öffnung (634) aufweist, die sich in einer axialen Mitte der Hautaufbringungsfläche der multifunktionalen Spitze (620) befindet, um mit dem Fluidabgabekanal (632) zu kommunizieren, der an dem hohlen Abschnitt der Antriebswelle (644) definiert ist, und Fluid an die Hautoberfläche abgibt;
wobei die Antriebswelle (644) die Doppelfunktion hat, die Hautaufbringungsfläche der multifunktionalen Spitze (620) zu drehen und gleichzeitig einen Strom eines Fluids durch den Fluidabgabekanal (632) zu der Hautaufbringungsfläche der multifunktionalen Spitze (620) an der Öffnung (634) zu führen;
wobei das zweite Fluidrohr (666) einen Vakuumeinlass (621) aufweist, wobei sich der Vakuumdurchgang (657) durch das Trägerelement (650) erstreckt, um ein verwendetes Fluid, das durch den Vakuumeinlass (621) gesammelt wird, von der multifunktionalen Spitze durch den Vakuumschluss (659) zu dem Fluidreservoir an einem hinteren Ende des Trägerelements (650) zu transportieren; und
ein Steuermodul (670) mit einem Steuerkreis (672), der betriebsmäßig mit den Elektroden (626) verbunden ist, um den Betrieb der Elektroden (626) und des Spitzenantriebs (640) zu steuern, wobei eine Übertragungseinheit (674) betriebsmäßig mit der Antriebseinheit (642) des Spitzenantriebs (640) verbunden ist, um die Amplitude der Antriebswelle (644) des Spitzenantriebs ( 640) anzupassen.

2. Die Vorrichtung nach Anspruch 1, wobei das Trägerelement (650) stationär ist und eine Bewegung der Antriebswelle (644) innerhalb der Mittelnut (652) des Trägerelements (650) nur in einer Richtung oder vor und zurück innerhalb der Mittelnut (650) des Trägerelements (650) einschränkt, wodurch jegliche unerwünschten Vibrationen der Antriebswelle (644) verhindert werden, wenn sie in Betrieb ist.

3. Die Vorrichtung nach Anspruch 1, wobei die Antriebswelle (644) beweglich ist und sich durch die Mittelnut des Trägerelements (650) erstreckt, um den Fluideinlass (636), der quer an der Antriebswelle (644) ausgebildet ist, so zu positionieren, dass, wenn die Antriebswelle (644) in Bezug auf das Trägerelement (650) bewegt wird, ein Fluid in den Fluideinlass (636) aus dem Fluidabgabekanal (658) eintreten kann.

4. Die Vorrichtung nach Anspruch 1, wobei das Trägerelement (650) ferner zwei Dichtungselemente (656) umfasst, die an einer Innenwand der Mittelnut (652) des Trägerelements (650) eingebettet sind, um den Fluideinlass (636) innerhalb des inneren Fluidhohlraums (654) zwischen den beiden Dichtungselementen (656) fluidisch abzudichten, um ein Auslaufen des Fluids innerhalb der Mittelnut (652) des Trägerelements (650) zu verhindern, wenn sich die Antriebswelle (644) bewegt.

5. Die Vorrichtung nach Anspruch 1, wobei sich der fluidführende Durchlass(658) von der Rückseite des Trägerelements (650) zu dem inneren Fluidhohlraum (654) erstreckt, wobei das Fluid so geführt wird, dass es von dem fluidführenden Durchlass(658) zu dem inneren Fluidhohlraum (654) fließt, bevor es so geführt wird, dass es von dem Fluideinlass (636) in den Fluidabgabekanal (632) fließt.

6. Die Vorrichtung nach Anspruch 1, wobei die in der axialen Mitte der Hautaufbringungsfläche ausgebildete Öffnung (634) eine Vielzahl von Fluidverteilungskanälen (628) definiert, die sich radial und nach außen von der Öffnung (634) zu den Elektroden (626) erstrecken, wobei jeder der Fluidverteilungskanäle (628) in einem Spalt zwischen zwei benachbarten Abriebelementen (622) ausgebildet ist.

7. Die Vorrichtung nach Anspruch 1, wobei die multifunktionale Spitze weiterhin eine Mikronadel-Anordnung mit einer Vielzahl von Mikronadeln umfasst, die an der Hautaufbringungsfläche vorgesehen sind.

8. Die Vorrichtung nach Anspruch 1, wobei der Fluidabgabekanal (632), der zwischen der Öffnung (634), die sich in der axialen Mitte der Hautaufbringungsfläche der multifunktionalen Spitze befindet, und dem Vakuumeinlass (621) definiert ist, einen Fluidstrom gewährleistet, der durch die Öffnung (634) auf die Hautaufbringungsfläche zugeführt wird und mit den Abriebelementen (622) und den Elektroden (626) in Wechselwirkung tritt, bevor das Fluid durch den Vakuumeinlass (621) über den Vakuumschluss (659) eingesaugt wird, so dass drei verschiedene Funktionen von Hautbehandlungen, nämlich abrasives Peeling, elektrische Stimulation und Flüssigkeitsinfusion in einem Schritt erreicht werden.

## Revendications

1. Equipement pour la distribution transdermique de fluide, ledit dispositif ayant une structure formant poignée
(610),
une pointe multifonctionnelle (620) avec une surface d'application cutanée, une structure de distribution de fluide (630) et un dispositif d'entraînement de pointe (640), l'équipement comprenant :
une enveloppe (612) à l'intérieur de ladite structure formant poignée (610) avec une extrémité de travail avant et une extrémité de communication arrière ;
ladite structure formant poignée (610) comprenant en outre une poignée (614) inclinée et s'étendant entre ladite extrémité de travail avant et ladite extrémité de communication arrière de ladite enveloppe (612) ;
un capuchon amovible (616) couplé à ladite enveloppe (612) à ladite extrémité de travail de ladite enveloppe (612) ;
un bord extérieur en forme de couronne (618) à une extrémité de travail dudit capuchon amovible (616) pour appliquer une pression sur une surface de peau pour effectuer des extractions sous pression ;
ladite pointe multifonctionnelle (620) avec ladite surface d'application cutanée située à l'intérieur dudit capuchon amovible (616) à ladite extrémité de travail de ladite enveloppe (612) à l'intérieur de ladite structure formant poignée (610) ;
dans lequel ledit dispositif d'entraînement de pointe (640) comprend, une unité d'entraînement (642) et un axe d'entraînement (644) ;
un élément formant support (650) à l'intérieur de ladite enveloppe (612) situé entre ladite unité d'entraînement (642) et ladite pointe multifonctionnelle (620) pour supporter ledit axe d'entraînement (644) dudit dispositif d'entraînement de pointe (640) ;
ledit élément formant support (650) ayant un passage de guidage de fluide (658) s'étendant depuis un côté arrière de l'élément formant support (650) jusqu'à une cavité intérieure à fluide (654), et en ce qu'un premier tube à fluide (662) s'étend depuis l'entrée du passage de guidage de fluide (658) et s'étend hors de l'extrémité de communication de l'enveloppe (612) pour relier fonctionnellement à la source de fluide ;
ledit élément formant support (650) ayant en outre un passage d'aspiration (657) s'étendant à travers l'élément formant support (650) et un orifice d'aspiration (659), et un second tube à fluide (666) qui s'étend depuis la sortie du passage d'aspiration (657) et s'étend hors de l'extrémité de communication de l'enveloppe (612) pour relier fonctionnellement à un réservoir de fluide ;
ledit élément formant support (650) ayant une fente centrale traversante (652) dans laquelle ledit axe d'entraînement (644) dudit dispositif d'entraînement de pointe (640) s'étend fonctionnellement depuis ladite unité d'entraînement (642) jusqu'à la pointe multifonctionnelle (620) ;
dans lequel ladite surface d'application cutanée de ladite pointe multifonctionnelle (620) comprend une pluralité d'éléments abrasifs (622) et un module formant électrode comprenant une pluralité d'électrodes (626) encerclant lesdits éléments abrasifs (622), avec ledit module formant électrode attaché à un côté intérieur dudit capuchon amovible (616) ;
dans lequel ledit élément formant support (650), ledit premier tube à fluide (662), ledit deuxième tube à fluide (666) et ledit dispositif d'entraînement de pointe (640) sont fixés à l'intérieur de ladite enveloppe (612) à l'intérieur de ladite structure formant poignée (610) ;
dans lequel ledit axe d'entraînement (644) s'étend fonctionnellement depuis ladite unité d'entraînement (642) jusqu'à ladite pointe multifonctionnelle (620) à travers ladite fente centrale traversante (652) dudit élément formant support (650), dans lequel ledit axe d'entraînement (644) est mobile et ledit élément formant support (650) est fixe ;
dans lequel ladite pointe multifonctionnelle (620) comprenant ladite pluralité d'éléments abrasifs (622) et ladite pluralité d'électrodes (626) sur ledit module formant électrode est couplée de manière amovible à une extrémité libre dudit axe d'entraînement (644) ;
dans lequel ledit axe d'entraînement (644) a au moins une partie creuse s'étendant jusqu'à ladite pointe multifonctionnelle, dans lequel un canal de distribution de fluide (632) de ladite structure de distribution de fluide (630) est défini à ladite partie creuse dudit axe d'entraînement (644) ;
dans lequel ladite structure de distribution de fluide (630) a en outre une entrée de fluide (636) formée transversalement audit axe d'entraînement (644) pour guider un fluide entrant dans ledit canal de distribution de fluide (632) depuis la cavité intérieure à fluide (654) et avec ladite cavité intérieure à fluide (654) située à l'intérieur dudit élément formant support (650) ;
dans lequel ledit canal de distribution de fluide (632) a au moins une ouverture (634) située à un centre axial de ladite surface d'application cutanée de ladite pointe multifonctionnelle (620) pour communiquer avec ledit canal de distribution de fluide (632) défini au niveau de ladite partie creuse dudit axe d'entraînement (644) et distribuer du fluide à la surface de la peau ;
dans lequel ledit axe d'entraînement (644) a une double fonction d'entraînement de ladite surface d'application cutanée de ladite pointe multifonctionnelle (620) pour que celle-ci tourne tout en guidant simultanément un écoulement d'un fluide à travers ledit canal de distribution de fluide (632) jusqu'à ladite surface d'application cutanée de ladite pointe multifonctionnelle (620) à ladite ouverture (634) ;
dans lequel ledit deuxième tube de fluide (666) comprend une entrée d'aspiration (621), dans lequel ledit passage d'aspiration (657) s'étend à travers ledit élément formant support (650) pour transporter un fluide usé collecté par ladite entrée d'aspiration (621) depuis ladite pointe multifonctionnelle par ledit orifice d'aspiration (659) vers le réservoir de fluide à une extrémité arrière dudit élément formant support (650) ; et
un module de commande (670) ayant un circuit de commande (672) connecté fonctionnellement aux électrodes (626) pour commander le fonctionnement des électrodes (626) et du dispositif d'entraînement de pointe (640) dans lequel une unité de transmission (674) est connectée fonctionnellement à ladite unité d'entraînement (642) dudit dispositif d'entraînement de pointe (640) pour ajuster l'amplitude dudit axe d'entraînement (644) dudit dispositif d'entraînement de pointe (640).

2. Equipement selon la revendication 1, dans lequel ledit élément formant support (650) est stationnaire et restreint un mouvement dudit axe d'entraînement (644) à l'intérieur de ladite fente centrale (652) dudit élément formant support (650) uniquement dans une direction ou selon un va-et-vient à l'intérieur de ladite fente centrale (650) dudit élément formant support (650) empêchant ainsi toute vibration indésirable dudit axe d'entraînement (644) lorsqu'il est en fonctionnement.

3. Equipement selon la revendication 1, dans lequel ledit axe d'entraînement (644) est mobile et s'étend à travers ladite fente centrale dudit élément formant support (650) pour disposer ladite entrée de fluide (636) formée transversalement audit axe d'entraînement (644) de sorte que, lorsque ledit axe d'entraînement (644) est déplacé par rapport audit élément formant support (650), un fluide peut entrer dans ladite entrée de fluide (636) depuis ledit canal de distribution de fluide (658).

4. Equipement selon la revendication 1, dans lequel ledit élément formant support (650) comprend en outre deux éléments d'étanchéité (656) incorporés sur une paroi interne de ladite fente centrale (652) dudit élément formant support (650) pour étanchéifier aux fluides ladite entrée de fluide (636) à l'intérieur de ladite cavité intérieure à fluide (654) entre lesdits deux éléments d'étanchéité (656) pour empêcher la fuite dudit fluide à l'intérieur de ladite fente centrale (652) dudit élément formant support (650) lorsque l'axe d'entraînement (644) se déplace.

5. Equipement selon la revendication 1, dans lequel ledit passage de guidage de fluide (658) s'étend depuis le côté arrière dudit élément formant support (650) jusqu'à ladite cavité intérieure à fluide (654), dans lequel ledit fluide est guidé pour s'écouler depuis ledit passage de guidage de fluide (658) jusqu'à ladite cavité intérieure à fluide (654) avant qu'elle ne soit guidée pour s'écouler dans ledit canal de distribution de fluide (632) depuis ladite entrée de fluide (636).

6. Equipement selon la revendication 1, dans lequel ladite ouverture (634) formée audit centre axial de ladite surface d'application cutanée définit une pluralité de canaux de répartition de fluide (628) s'étendant radialement et vers l'extérieur depuis ladite ouverture (634) jusqu'aux dites électrodes (626) dans lequel chacun lesdits canaux de répartition de fluide (628) est formé en un espace entre deux éléments abrasifs adjacents (622).

7. Equipement selon la revendication 1, dans lequel ladite pointe multifonctionnelle comprend en outre un ensemble à micro-aiguilles ayant une pluralité de micro-aiguilles prévues sur ladite surface d'application cutanée.

8. Equipement selon la revendication 1, dans lequel ledit canal de distribution de fluide (632) défini entre ladite ouverture (634) située au niveau dudit centre axial de ladite surface d'application cutanée de ladite pointe multifonctionnelle et de ladite entrée d'aspiration (621) assure un écoulement de fluide devant être distribué sur ladite surface d'application cutanée à travers ladite ouverture (634) et pour interagir avec lesdits éléments abrasifs (622) et lesdites électrodes (626) avant que ledit fluide ne soit retiré par ladite entrée d'aspiration (621) à travers ledit orifice d'aspiration (659), de sorte que trois fonctions différentes de traitements de la peau, à savoir : une desquamation abrasive, une stimulation électrique et une infusion de liquide, sont obtenues en une seule étape.
